# EUROPÄISCHE PATENTSCHRIFT

(11) **EP 0 595 125 B1**
(45) Veröffentlichungstag und Bekanntmachung des Hinweises auf die Patenterteilung: **15.05.1996**
(21) Anmeldenummer: 93116618.5
(22) Anmeldetag: 14.10.1993
(51) Int. Cl.: C07D 213/24, A01N 43/40, C07D 213/26, C07D 213/30, A01N 43/78, C07D 277/32, C07D 401/06, C07D 413/06

(54) **Substituierte Aza(cyclo) Alkane und ihre Verwendung als Schadlingsbekampfungsmittel**
Substituted aza(cyclo) alkanes and their use as pesticides
Aza(cyclo) alcanes substitués et leur utilisation comme pesticides

(30) Priorität: 27.10.1992 DE 4236204
(43) Veröffentlichungstag der Anmeldung: 04.05.1994
(73) Patentinhaber: BAYER AG, D-51368 Leverkusen (DE)
(72) Erfinder: Wagner, Klaus, Dr., D-51065 Köln (DE); Erdelen, Christoph, Dr., D-42799 Leichlingen (DE); Hartwig, Jürgen, Dr., D-42799 Leichlingen (DE); Leicht, Wolfgang, Dr., D-51371 Leverkusen (DE); Stendel, Wilhelm, Dr., D-42113 Wuppertal (DE)

(56) Entgegenhaltungen:
- EP-A- 0 192 060
- EP-A- 0 386 565
- EP-A- 0 425 978
- EP-A- 0 455 000
- EP-A- 0 483 052
- EP-A- 0 547 557
- US-A- 4 849 432

## Beschreibung

Die Erfindung betrifft neue substituierte Aza(cydo)alkane, Verfahren zu ihrer Herstellung und ihre Verwendung als Schädlingsbekämpfungsmittel, insbesondere als Insektizide.

Es ist bereits bekannt, daß bestimmte heterocyclische Verbindungen insektizide Eigenschaften aufweisen (vgl. EP-A 192 060, EP-A 483 052, EP-A 455 000, EP-A 425 978, EP-A 386 565, US-A 4 849 432).

Es wurden nun die neuen substituierten Aza(cyclo)alkane der allgemeinen Formel (I) gefunden, 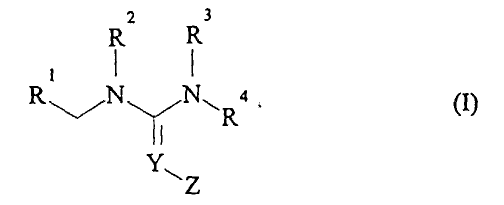 in welcher
- R¹: für eine fünf- bis sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl, welche durch Fluor, Chrom, Brom, Iod, Cyano, Nitro, C₁-C₄-Alkyl (welches gegebenenfalls durch Fluor und oder Chlor substituiert ist), C₂-C₄-Alkenyl (welches gegebenenfalls durch Fluor und/oder Chlor substituiert ist), C₂-C₄-Alkinyl, C₁-C₄-Alkoxy (welches gegebenenfalls durch Fluor und/oder Chlor substituiert ist), C₃-C₄-Alkenyloxy (welches gegebenenfalls durch Fluor und/oder Chlor substituiert ist), C₃-C₄-Alkinyloxy, C₃-C₄-Alkylthio (welches gegebenenfalls durch Fluor und/oder Chlor substituiert ist), C₃-C₄-Alkenylthio (welches gegebenenfalls durch Fluor und/oder Chlor substituiert ist), C₃-C₄-Alkinylthio, C₁ C₄-Alkylsulfinyl (welches gegebenenfalls durch Fluor und/oder Chlor substituiert ist), C₁-C₄-Alksulfonyl (welches gegenenenfalls durch Fluor und /oder Chlor substituiert ist), Amino, C₁-C₄-Alkylamino, Di-(C₁-C₄-alkyl)-amino, Phenyl, Phenoxy, Phenylthio, Phenylamino, Benzyl, Formylamino, C₁-C₄-Alkyl-carbonylamino, Formyl, Carbamoyl, C₁-C₄-Alkylcarbonyl und/oder C₁-C₄-Alkoxy-carbonyl substituiert ist, steht
- R: für C₁-C₆-Hydroxyalkyl, Dihydroxy-C₁-C₆-alkyl, C₁-C₄-Alkoxy-C₁-C₄-alkyl, Di-(C₁-C₄-alkoxy)-C₁-C₄-alkyl steht,
- R³: für Wasserstoff, C₁-C₆-Alkyl, C₁-C₆-Hydroxyalkyl, Dihydroxy-C₁-C₆-alkyl, C₁-C₄-Alkoxy-C₁-C₄-alkyl, Di-(C₁-C₄-alkoxy)-C₁-C₄-alkyl oder
- R und R³: zusammen für Hydroxy-C₂-C₄-alkandiyl, Dihydroxy-C₂-C₄-alkandiyl, C₁-C₄-Alkoxy-C₂-C₄-alkandiyl, Di-(C₁-C₄-alkoxy)-C₂-C₄-alkandiyl, Oxo-C₂-C₄-alkandiyl oder Dioxo-C₂-C₄-alkandiyl stehen,
- R⁴: für Wasserstoff, C₁-C₄-Alkyl (welches gegebenenfalls durch Fluor, Chlor , Cyano, C₁-C₄-Alkoxy, C₁-C₄-Akylthio, Di-(C₁-C₄-alkyl)-amino. Trimethylsilyl, C₁-C₄--Alkoxy-carbonyl, Carboxy, Carbamoyl, C₁-C₄-Alkyl-aminocarbonyl, Di-(C₁-C₃-alkyl)-aminocarbonyl, oder durch eine heterocyclische Gruppierung, wie sie oben für R¹ (einschließlich der möglichen Substituenten) vorzugsweise definiert ist, substituiert ist), C₂-C₄-Alkenyl (welches gegebenenfalls durch Fluor oder Chlor substituiert ist), C₂-C₄-Alkinyl, Benzyl (welches gegebenenfalls durch Fluor, Chlor, Cyano, Nitro, C₁-C₂-Alkyl, Trifluormethyl, C₁-C₂-Alkoxy oder C₁-C₂-Alkoxy-carbonyl substituiert ist), Formyl, C₁-C₂₀-Alkyl-carbonyl (welches gegebenenfalls durch Fluor, Chlor, Brom, Phenyl, Phenoxy oder C₁-C₄-Alkoxy substituiert ist), C₃-C₆-Cycloalkylcarbonyl (welches gegebenenfalls durch Fluor, Chlor und/oder C₁-C₄-Alkyl substituiert ist), C₂-C₂₀-Alkenyl-carbonyl (welches gegebenenfalls durch Fluor und/oder Chlor substituiert ist), Phenylcarbonyl oderNaphthylcarbonyl (welche gegebenenfalls durch Fluor, Chlor, Brom, C₁-C₄-Alkyl, Trifluormethyl, Cyano, Nitro, C₁-C₄-Alkoxy und/oder C₁-C₄-Alkoxy-carbonyl substituiert sind), C₁-C₂₀-Alkoxy-carbonyl, Benzyloxy-carbonyl, Phenoxyarbonyl, C₁-C₄-Alkylthiocarbonyl, Benzylthio-carbonyl, Phenylthio-carbonyl, C₁-C₆-Alkylamino-carbonyl, Di-(C₁-C₄-alkyl)-amino-carbonyl, Phenylamino-carbonyl (welches gegebenenfalls durch Fluor, Chlor, Brom, Cyano, Nitro, C₁-C₄-Alkyl, Trifluormethyl, C₁-C₄-Alkoxy, C₁-C₂-Fluoralkoxy, C₁-C₂-Chlor-fluoralkoxy, C₁-C₄-Alkylthio, C₁-C₂-Fluoralkylthio, C₁-C₂-Chlorfluoralkylthio oder C₁-C₄-Alkoxycarbonyl substituiert ist), Benzoylamino-carbonyl (welches gegebenenfalls durch Fluor, Chlor, Brom, Methyl oder Trifluormethyl substituiert ist), Phenylsulfonylamino-carbonyl (welches gegebenenfalls durch Fluor, Chlor, Brom, Methyl, Trifluormethyl, C₁-C₄-Alkoxy, C₁-C₂-Fluoralkoxy, C₁-C₂-Chlorfluoralkoxy oder C₁-C₄-Alkoxy-carbonyl substituiert ist), C₁-C₄-Akylthio (welches gegebenenfalls durch Fluor und/oder Chlor substituiert ist), Phenylthio (welches gegebenenfalls durch Fluor, Chlor, Brom, Nitro oder Methyl substituiert ist), C₁-C₄-Alkylsulfinyl, C₁-C₄-Alkylsulfonyl (welches gegebenenfalls durch Fluor und/oder Chlor substituiert ist), Phenylsulfinyl (welches gegebenenfalls durch Fluor, Chlor, Brom, Nitro oder Methyl substituiert ist), Phenylsulfonyl oder Naphthylsulfonyl (welche gegebenenfalls durch Fluor, Chlor, Brom, Cyano, Nitro, Methyl, Trifluormethyl, C₁-C₄-Alkoxy, C₁-C₂-Fluoralkoxy, C₁-C₂-Chlorfluoralkoxy und/oder C₁-C₄-Alkoxy-carbonyl substituiert sind), Dimethyl(thio)phosphoryl, C₁-C₄-Alkyl-C₁-C₄-alkoxy-(thio)phosphoryl oder Di(C₁-C₄-alkoxy)-(thio)phosphoryl steht,
- Y: für Stickstoff oder eine CH-Gruppierung steht und
- Z: für Cyano oder Nitro steht.

Man erhält die neuen substituierten Aza(cyclo)alkane der allgemeinen Formel (I), wenn man
(a) Azaalkane der allgemeinen Formel (II) 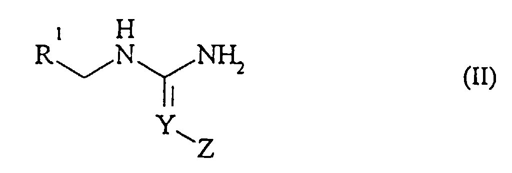 in welcher
   - R¹, Y und Z: die oben angegebene Bedeutung haben,
   mit Halogenverbindungen der allgemeinen Formel (IIIa)

   X-R (IIIa)

   und/oder Halogenverbindungen der allgemeinen Formel (IIIb)

   X-R³ (IIIb)

   worin
   - R und R³: die oben angegebene Bedeutung haben und
   - X: für Halogen steht,
   gegebenenfalls in Gegenwart eines Säureakzeptors, gegebenenfalls in Gegenwart eines Katalysators oder gegebenenfalls in Gegenwart eines Verdünnungsmittels umsetzt,
   oder wenn man
(b) Verbindungen der allgemeinen Formel (I), in welcher R oder R³ für Dialkoxyalkyl steht und R¹, R⁴, Y und Z die oben angegebene Bedeutung haben,
   gegebenenfalls in Gegenwart eines Reaktionshilfsmittels und gegebenenfalls in Gegenwart eines Verdünnungsmittels erhitzt,
   oder wenn man
(c) Verbindungen der allgemeinen Formel (I), in welcher R und R³ zusammen für Oxoalkandiyl stehen und R¹, R⁴, Y und Z die oben angegebene Bedeutung haben,
   mit einem Hydrierungsmittel, gegebenenfalls in Gegenwart eines Reaktionshilfsmittels und gegebenenfalls in Gegenwart eines Verdünnungsmittels umsetzt,
   oder wenn man
(d) Verbindungen der allgemeinen Formel (I), in welcher R und R³ zusammen für Alkoxyalkandiyl odere Dialkoxyalkandiyl stehen und R¹, R⁴, Y und Z die oben angegebene Bedeutung haben,
   mit einem Hydrogenhalogenid und/oder einem Alkalimetallhalogenid sowie in Gegenwart von Verdünnungsmitteln umsetzt,
   oder wenn man
(e) Verbindungen der allgemeinen Formel (I), in welcher R und R³ zusammen für Hydroxyalkandiyl oder Dihydroxyalkandiyl stehen und R¹, R⁴, Y und Z die oben angegebene Bedeutung haben,
   mit einem Alkylierungsmittel der allgemeinen Formel (IV)

   X¹-R (IV)

   in welcher
   - R: für Alkyl steht und
   - X¹: für Halogen oder die Gruppierung -O-SO₂-O-R steht, worin R die oben angegebene Bedeutung hat,
   gegebenenfalls in Gegenwart eines Säurebindemittels und gegebenenfalls in Gegenwart eines Verdünnungsmittels umsetzt,
   oder wenn man
(f) Verbindungen der allgemeinen Formel (I), in welcher R⁴ für Wasserstoff steht und R¹, R, R³, Y und Z die oben angegebene Beutung haben, mit Halogenverbindungen der allgemeinen Formel (V)

   X-R⁴ (V)

   in welcher
   - X: für Halogen steht und
   - R⁴: mit Ausnahme von Wasserstoff die oben angegebene Bedeutung hat,
   gegebenenfalls in Gegenwart eines Säureakzeptors und gegebenenfalls in Gegenwart eines Verdünnungsmittels umsetzt.

Die neuen substituierten Aza(cyclo)alkane der allgemeinen Formel (I) zeichnen sich durch hohe Wirksamkeit als Insektizide aus.

Die Erfindung betrifft vorzugsweise Verbindungen der Formel (I), in welcher
- R¹: für eine fünf- bzw. sechsgliedrige heterocyclische Gruppierung aus der Reihe Pyrazoyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert ist,
- R: für C₁-C₄-Hydroxyalkyl, Dihydroxy-C₁-C₄-alkyl, C₁-C₃-Alkoxy-C₁-C₃-alkyl, Di-(C₁-C₃-alkoxy)-C₁-C₃-alkyl,
- R³: für Wasserstoff, C₁-C₄-Alkyl, C₁-C₄-Hydroxyalkyl, Dihydroxy-C₁-C₄-alkyl, C₁-C₃-Alkoxy-C₁-C₃-alkyl, Di-(C₁-C₃-alkoxy)-C₁-C₃-alkyl oder
- R und R³: zusammen für Hydroxy-C₂-C₃-alkandiyl, Dihydroxy-C₂-C₃-alkandiyl, C₁-C₃-Alkoxy-C₂-C₃-alkandiyl, Di-(C₁-C₃-alkoxy)-C₂-C₃ -alkandiyl, Oxo-C₂-C₃-alkandiyl oder Dioxo-C2-C3-alkandiyl stehen,
- R⁴: für Wasserstoff, Methyl, Ethyl, Allyl, Propargyl, Formyl, C₁-C₈-Alkylcarbonyl (welches gegebenenfalls durch Fluor und/oder Chlor substituiert ist), C₁-C₈-Alkoxy-carbonyl, Benzyloxy-carbonyl, Phenoxycarbonyl, Benzyl (welches gegebenenfalls durch Fluor oder Chlor substituiert ist) oder-Di-(C₁-C₂-alkoxy)-(thio)phosphoryl steht,
- Y: für Stickstoffoder eine CH-Gruppierung steht und
- Z: für Cyano oder Nitro steht.

Ganz besonders bevorzugt sind die Verbindungen der Formel (1), in welcher
- R¹: für 6-Chlor-3-pyridyl (6-Chlor-pyridin-3-yl) oder für 2-Chlor-5-thiazolyl (2-Chlor-thiazol-5-yl) steht,
- R: für Hydroxymethyl, Hydroxyethyl, Hydroxypropyl, Dihydroxyethyl, Dihydroxypropyl, Methoxymethyl, Ethoxymethyl, Methoxyethyl, Ethoxyethyl, Dimethoxyethyl, Diethoxyethyl,
- R³: für Wasserstoff, Methyl Ethyl, Hydroxymethyl, Hydroxyethyl, Hydroxypropyl, Dihydroxyethyl, Dihydroxypropyl, Methoxymethyl, Ethoxymethyl, Methoxyethyl, Ethoxyethyl, Dimethoxyethyl, Diethoxyethyl,
- R und R³: zusammen für Oxoethan-1,2-diyl, Dioxoethan-1,2-diyl, Hydroxyethan-1,2-diyl, Dihydroxyethan-1,2-diyl, Methoxyethan-1,2-diyl, Ethoxyethan-1,2-diyl, Dimethoxyethan-1,2-diyl oder Diethoxyethan-1,2-diyl stehen,
- R⁴: für Wasserstoff oder Methyl steht,
- Y: für Stickstoff oder eine CH-Gruppierung steht und
- Z: für Cyano oder Nitro steht.

Die oben bei der Definition der erfindungsgemäßen Verbindungen der allgemeinen Formel (I) genannten Kohlenwasserstoffreste wie Alkyl sind - auch in Verbindung mit Heteroatomen wie in Alkoxy - soweit möglich jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die oben aufgeführten allgemeinen oder im Vorzugsbereichen aufgeführten Restdefinitionen bzw. Erläuterungen gelten für die Endprodukte der Formel (I) wie auch für die Ausgangsstoffe und Zwischenprodukte entsprechend.

Die Restdefinitionen können untereinander, also auch zwischen den jeweiligen Vorzugsbereichen beliebig kombiniert werden.

Beispiele für die Verbindungen der Formel (I) sind in der nachstehenden Tabelle 1 aufgeführt.

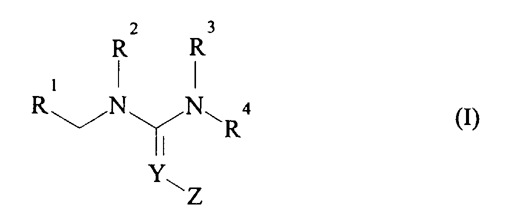

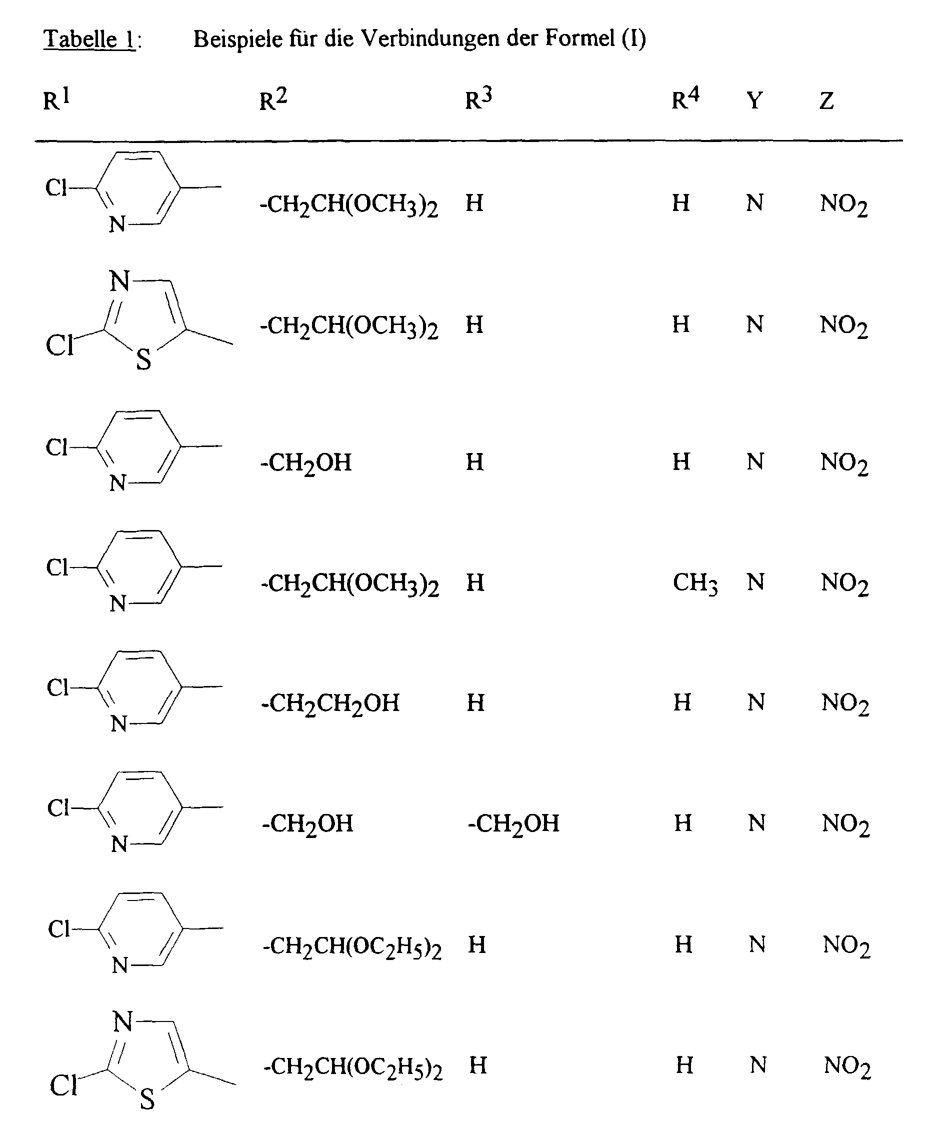

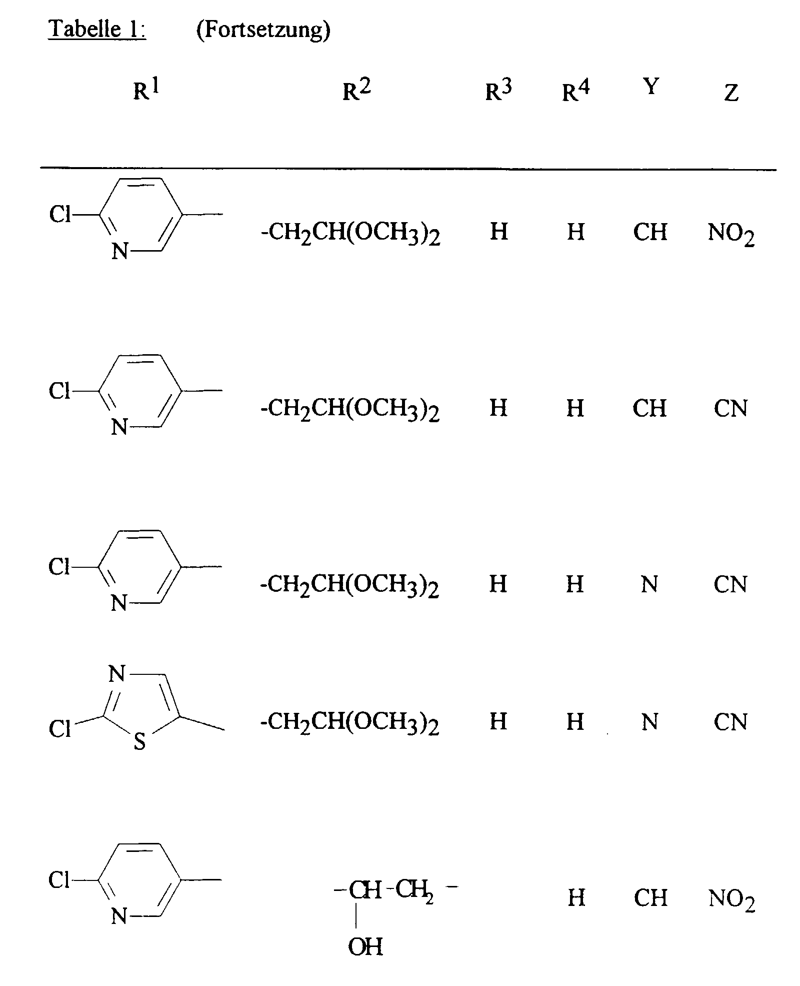

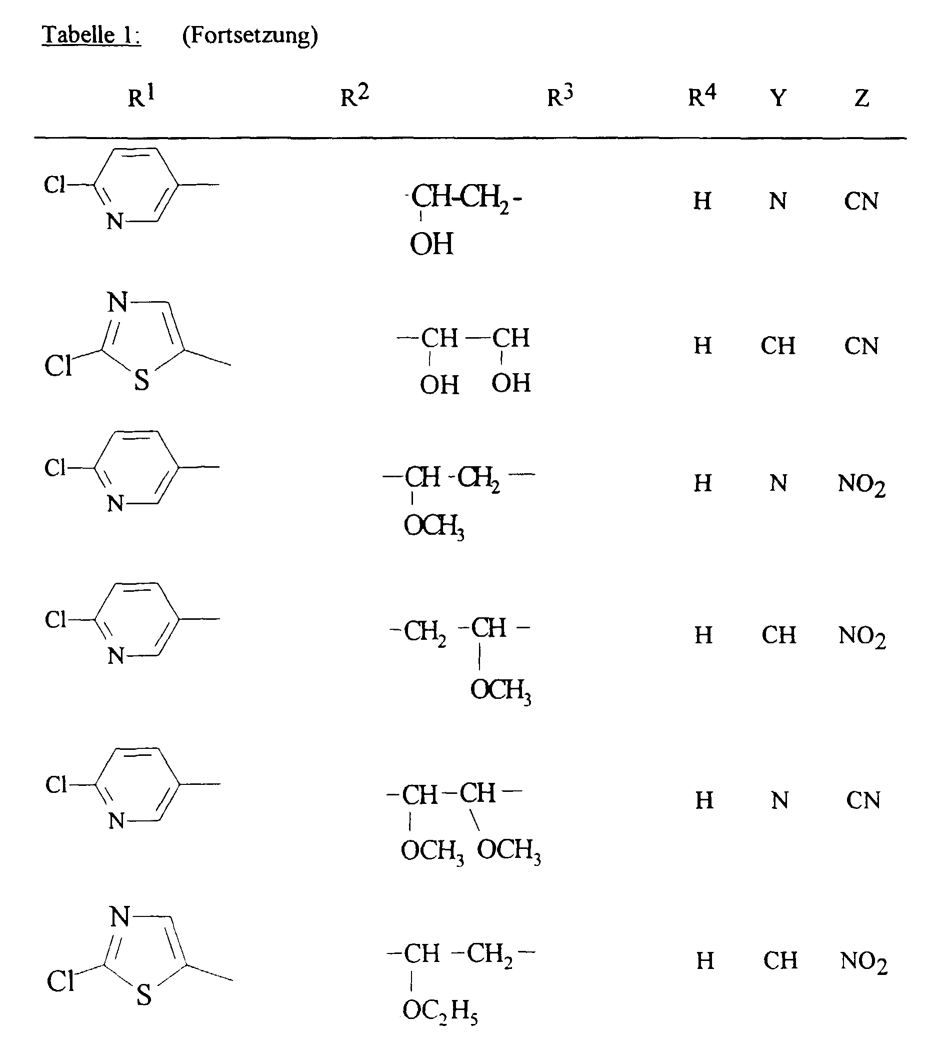

Verwendet man beispielsweise 1- (2-Thiazol-5-yl-methyl) -2-cyano-guanidin und Chloracetaldehyd-dimethylacetal als Ausgangsstoffe, so kann der Reaktionsablauf beim erfindungsgemäßen Verfahren (a) durch das folgende Formelschema wiedergegeben werden:

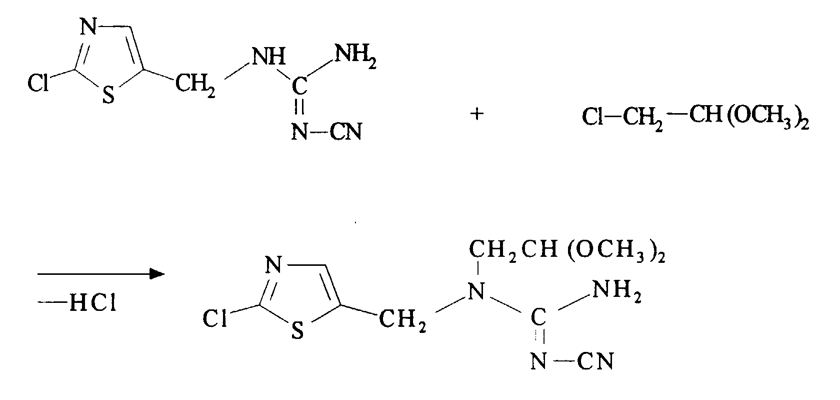

Verwendet man beispielsweise 1-(6-Chlor-pyridin-3-yl-methyl)-1-(2,2-dimethoxyethyl)-2-nitro-guanidin als Ausgangsstoff, so kann der Reaktionsablauf beim erfindungsgemäßen Verfahren (b) durch das folgende Formelschema wiedergegeben werden:

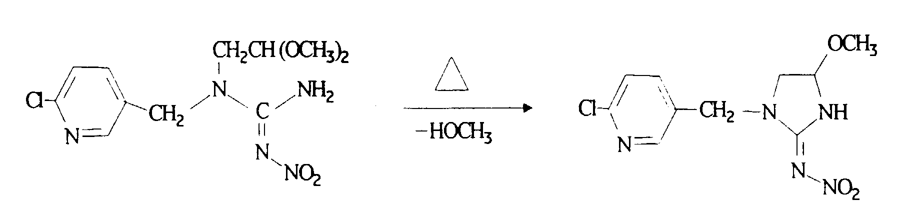

Verwendet man beispielsweise 1-(2-Chlor-thiazol-5-yl-methyl)-2-nitromethylen-5-oxo-imidazolidin und Natriumboranat als Ausgangsstoffe, so kann der Reaktionsablauf beim erfindungsgemäßen Verfahren (c) durch das folgende Formelschema wiedergegeben werden:

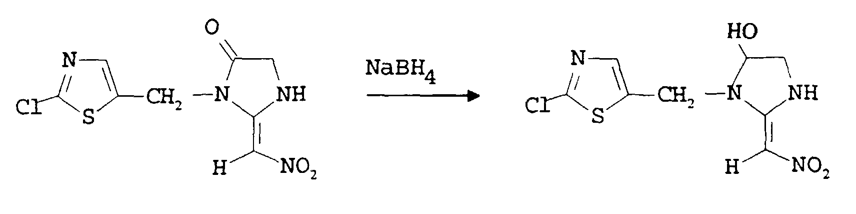

Verwendet man beispielsweise 1-(6-Chlor-pyridin-3-yl-methyl)-2-cyanomethylen-4-methoxy-imidazolidin und Hydrogenbromid als Ausgangsstoffe, so kann der Reaktionsablauf beim erfindungsgemäßen Verfahren (d) durch das folgende Formelschema wiedergegeben werden:

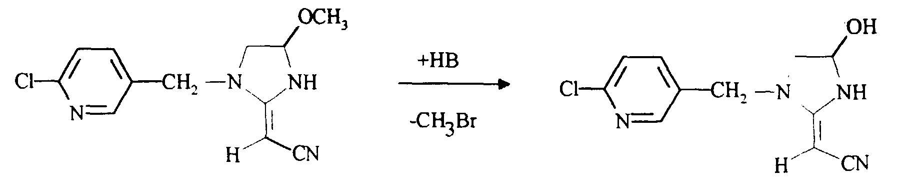

Verwendet man beispielsweise 1-(6-Chlor-pyridin-3yl-methyl)-2-nitroimino-3-ethyl-5-hydroxy-imidazolidin und Ethylbromid als Ausgangsstoffe, so kann der Reaktionsablauf beim erfindungsgemäßen Verfahren (e) durch das folgende Formelschema wiedergegeben werden:

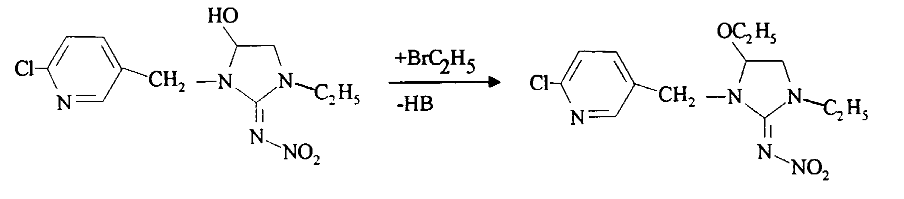

Verwendet man beispielsweise 1-(6-Chlor-pyridin-3-yl-methyl)-1-(2,2-dimethoxyethyl)-2-nitro-guanidin und Methylbromid als Ausgangsstoffe, so kann der Reaktionsablauf beim erfindungsgemäßen Verfahren (f) durch das folgende Formelschema wiedergegeben werden:

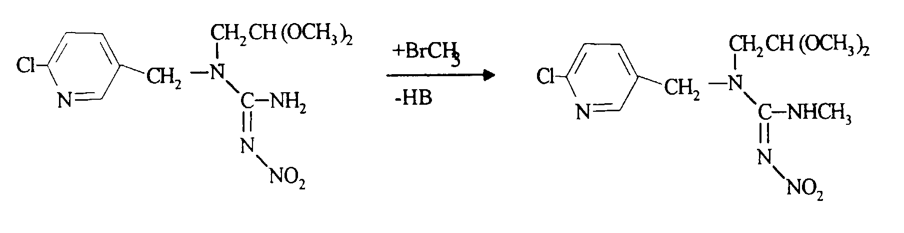

Die beim erfindungsgemäßen Verfahren (a) zur Herstellung von Verbindungen der Formel (I) als Ausgangsstoffe zu verwendenden Azoalkane sind durch die Formel (II) allgemein definiert.

In Formel (II) haben R¹, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, Y und Z angegeben wurden.

Die Ausgangsstoffe der Formel (II) sind bekannt und/oder können nach an sich bekannten Verfahren hergestellt werden (vergleiche EP-A 302389, EP-A 306696, EP-A 364844, EP-A 375907, EP-A 376297, EP-A 381130, EP-A 418199, EP-A 425978, EP-A 428941, EP-A 452782).

Die beim erfindungsgemäßen Verfahren (a) zur Herstellung von Verbindungen der Formel (I) weiter als Ausgangsstoffe zu verwendenden Halogenverbindungen sind durch die Formel (IIIa) bzw. die Formel (IIIb) allgemein definiert.

In Formel (IIIa) bzw. Formel (IIIb) haben Rbzw. R³ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R bzw. R³ angegeben wurden; X steht jeweils vorzugsweise für Chlor oder Brom.

Die Ausgangsstoffe der Formeln (IIIa) und (IIIb) sind bekannte organische Synthesechemikalien.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetallhydroxide, wie Lithium-, Natrium-, Kalium- und Calcium-hydroxid, Alkalimetall- und Erdalkalimetall-carbonate und -hydrogencarbonate, wie Natrium- und Kaliumcarbonat oder Hydrogencarbonat sowie Calciumcarbonat, Alkalimetallacetate, wie Natrium- und Kalium-acetat, Alkalimetallalkoholate, wie Natrium- und Kaliummethylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, Ethyldiisopropylamin, Ethyldicyclohexylamin, N,N-Dimethylbenzylamin, N,N-Dimethyl-analin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-Methyl-pyridin, 1,5-Diazabicyclo-[ 4,3,0]-non-5-en (DBN), 1,8-Diazabicyclo-[5,4,0]-undec-7-en (DBU) und 1,4-Diazabicyclo-[2,2,2]-octan (DABCO).

Das erfindungsgemäße Verfahren (a) wird gegebenenfalls in Gegenwart eines Katalysators durchgeführt. Geeignete Katalysatoren sind hierbei insbesondere Alkalimetallsalze wie Kaliumchlorid, Rubidiumchlorid und Cäsiumchlorid.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen 0°C und 200°C, vorzugsweise bei Temperaturen zwischen 20°C und 150°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors und gegebenenfalls in Gegenwart eines Katalysators durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vergleiche die Herstellungsbeispiele).

Die beim erfindungsgemäßen Verfahren (b) als Ausgangsstoffe zu verwendenden Verbindungen sind durch die Formel (I) mit der Maßgabe, daß R oder R³ für Dialkoxyalkyl steht, allgemein definiert. In diesem Fall haben die Reste R¹, R, R³, R⁴, Y und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Die oben beschriebenen Ausgangsstoffe der Formel (I) für Verfahren (b) sind erfindungsgemäße neue Verbindungen; sie können nach dem erfindungsgemäßen Verfahren (a) hergestellt werden.

Das erfindungsgemäße Verfahren (b) wird gegebenenfalls in Gegenwart eines Reaktionshilfsmittels durchgeführt. Als Reaktionshilfsmittel sind hierbei insbesondere Lewis-Säuren wie Bortrifluorid oder Aluminiumtrichlorid, aber auch Mineralsäuren wie Salzsäure, Schwefelsäure, Methansulfonsäure, Benzolsulfonsäure und p-Toluolsulfonsäure geeignet.

Das erfindungsgemäße Verfahren (b) wird vorzugsweise unter Verwendung eines Verdünnungsmittels durchgeführt. Es kommen hierbei diejenigen Verdünnungsmittel in Betracht, die bereits bei der Beschreibung des erfindungsgemäßen Verfahrens (a) genannt wurden. Ferner können vorteilhalt auch Wasser und Alkohole wie Methanol, Ethanol, n- oder i- Propanol, n-, i-, s- oder t- Butanol als Verdünnungsmittel bei Verfahren (b) verwendet werden.

Die Reaktionstemperaturen können bei dem erfindungsgemäßen Verfahren (b) in einem größeren Bereich variiert werden. Im allgemeinen arbeitet man bei Temperaturen zwischen 20°C und 150°C, vorzugsweise bei Temperaturen zwischen 40°C und 100°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die Reaktionskomponenten im allgemeinen bei Raumtemperatur vermischt und dann bei der erforderlichen Temperatur bis zum Ende der Umsetzung gerührt. Die Aufarbeitung kann dann nach üblichen Methoden erfolgen (vergleiche die Herstellungsbeispiele).

Die beim erfindungsgemäßen Verfahren (c) als Ausgangsstoffe zu verwendenden Verbindungen sind durch die Formel (I) mit der Maßgabe, daß R und R³ zusammen für Oxoalkandiyl stehen, allgemein definiert. In diesem Fall haben die Reste R¹, R, R³, R⁴, Y und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Die oben beschriebenen Ausgangsstoffe der Formel (I) für Verfahren (c) sind erfindungsgemäße neue Verbindungen; sie können nach dem erfindungsgemäßen Verfahren (a) hergestellt werden.

Das erfindungsgemäße Verfahren (c) wird unter Verwendung eines Hydrierungsmittels durchgeführt. Es kommen hierbei insbesondere die zur Hydrierung von Carbonylverbindungen zu Hydroxyverbindungen geeigneten Metallhydridkomplexe wie Lithiumborhydrid (LiBH₄), Lithiumaluminiumhydrid (LiAlH₄) und Natriumborhydrid (NaBH₄) in Betracht.

Verfahren (c) wird gegebenenfalls in Gegenwart eines Reaktionshilfsmittels durchgeführt. Als Reaktionshilfsmittel sind hierbei insbesondere Säuren wie Salzsäure, Schwefelsäure oder Essigsäure geeignet.
Verfahren (c) wird vorzugsweise unter Verwendung eines Verdünnungsmittels durchgeführt. Es kommen hierbei diejenigen Verdünnungsmittel vorzugsweise in Betracht, die bereits bei der Beschreibung des erfindungsgemäßen Verfahrens (a) genannt wurden. Ferner können vorteilhaft auch Wasser, Alkohole wie Methanol, Ethanol, n-und i- Propanol und ferner auch Carbonsäuren wie Essigsäure und Propionsäure als Verdünnungsmittel bei Verfahren (c) verwendet werden.

Die Reaktionstemperaturen können bei dem erfindungsgemäßen Verfahren (c) in einem größeren Bereich variiert werden. Im allgemeinen arbeitet man bei Temperaturen zwischen -20°C und +50°C, vorzugsweise bei Temperaturen zwischen 0°C und + 30°C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Reaktionshilfsmittels durchgeführt, und das Reaktionsgemisch wird bei der jeweils erforderlichen Temperatur gerührt. Die Aufarbeitung erfolgt bei dem erfindungsgemäßen Verfahren (c) jeweils nach ublichen Methoden (vergleiche die Herstellungsbeispiele).

Die beim erfindungsgemäßen Verfahren (d) als Ausgangsstoffe zu verwendenden Verbindungen sind durch die Formel (I) mit der Maßgabe, daß R und R³ zusammen für Akoxyalkandiyl oder Dialkoxyalkandiyl stehen, allgemein definiert. In diesem Fall haben die Reste R¹, R, R³, R⁴, Y und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Die oben beschriebenen Ausgangsstoffe der Formel (I) für Verfahren (d) sind erfindungsgemäße neue Verbindungen; sie können nach den erfindungsgemäßen Verfahren (a) oder (b) hergestellt werden.

Das erfindungsgemäße Verfahren (d) wird in Gegenwart eines Hydrogenhalogenids und/oder eines Alkalimetallhalogenids durchgeführt. Vorzugsweise werden Hydrogenchlorid oder Hydrogenbromid und/oder entsprechende Natrium- oder Kaliumhalogenide, das heißt Natrium- oder Kalium-chlorid bzw. -bromid, eingesetzt.

Verfahren (d) wird vorzugsweise unter Verwendung eines Verdünnungsmittels durchgeführt. Es kommen hierbei neben Wasser vorzugsweise diejenigen Verdünnungsmittel in Betracht, die bereits bei der Beschreibung des erfindungsgemäßen Verfahrens (a) genannt wurden.

Die Reaktionstemperaturen können bei dem erfindungsgemäßen Verfahren (d) in einem größeren Bereich variiert werden. Im allgemeinen arbeitet man bei Temperaturen zwischen 0°C und 100°C, vorzugsweise bei Temperaturen zwischen 20°C und 80°C.

Das erfindungsgemäße Verfahren (d) wird im allgemeinen unter Normaldruck durchgeführt. Es ist jedoch auch möglich, unter erhöhtem oder vermidertem Druck zu arbeiten.

Zur Durchführung des erfindungsgemäßen Verfahrens (d) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (d) jeweils nach üblichen Methoden (vergleiche die Herstellungsbeispiele).

Die beim erfindungsgemäßen Verfahren (e) als Ausgangsstoffe zu verwendenden Verbindungen sind durch die Formel (I) mit der Maßgabe, daß R und R³ zusammen für Hydroxyalkandiyl oder Dihydroxyalkandiyl stehen, allgemein definiert. In diesem Fall haben die Reste R¹, R, R³, R⁴, Y und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Die oben beschriebenen Ausgangsstoffe der Formel (I) für Verfahren (e) sind erfindungsgemäße neue Verbindungen; sie können nach den erfindungsgemäßen Verfahren (c) oder (d) hergestellt werden.

Die beim erfindungsgemäßen Verfahren (e) zur Herstellung von Verbindungen der Formel (I) weiter als Ausgangsstoffe zu verwendenden Alkylierungsmittel sind durch die Formel (IV) allgemein definiert.

In Formel (IV) stehen
- R: vorzugsweise für C₁-C₄- Alkyl, insbesondere für Methyl oder Ethyl, und
- X: vorzugsweise für Chlor, Brom oder Iod oder für die Gruppierung -O-SO₂-O-R, worin R die oben angegebene Bedeutung hat.

Die Ausgangsstoffe der Formel (IV) sind bekannte organische Synthesechemikalien.

Das erfindungsgemäße Verfahren (e) wird vorzugsweise in Gegenwart eines Säureakzeptors durchgeführt. Es kommen hierbei vorzugsweise diejenigen Säurebindemittel in Betracht, die oben bei der Beschreibung des erfindungsgemäßen Verfahrens (a) angegeben wurden.

Das erfindungsgemäße Verfahren (e) wird vorzugsweise unter Verwendung eines Verdünnungsmittels durchgeführt. Es kommen hierbei vorzugsweise diejenigen Verdünnungsmittel in Frage, die oben bei der Beschreibung des erfindungsgemäßen Verfahrens (a) angegeben wurden.

Die Reaktionstemperaturen können bei dem erfindungsgemäßen Verfahren (e) in einem größeren Bereich variiert werden. Im allgemeinen arbeitet man bei Temperaturen zwischen 0°C und 150°C, vorzugsweise bei Temperaturen zwischen 20°C und 100°C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (e) jeweils nach üblichen Methoden (vergleiche die Herstellungsbeispiele).

Die beim erfindungsgemäßen Verfahren (f) als Ausgangsstoffe zu verwendenden Verbindungen sind durch die Formel (I) mit der Maßgabe, daß R⁴ für Wasserstoff steht, allgemein definiert. In diesem Fall haben die Reste R¹, R, R³, Y und Z vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden.

Die oben beschriebenen Ausgangsstoffe der Formel (I) für Verfahren (f) sind erfindungsgemäße neue Verbindungen; sie können nach den erfindungsgemäßen Verfahren (a) bis (d) hergestellt werden.

Die beim erfindungsgemäßen Verfahren (f) zur Herstellung von Verbindungen der Formel (I) weiter als Ausgangsstoffe zu verwendenden Halogenverbindungen sind durch die Formel (V) allgemein definiert.

In Formel (V) hat R⁴ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R⁴ angegeben wurde;
X steht vorzugsweise für Fluor, Chlor, Brom oder Iod, insbesondere für Chlor oder Brom.

Die Ausgangsstoffe der Formel (V) sind bekannte organische Synthesechemikalien.

Das erfindungsgemäße Verfahren (f) wird vorzugsweise in Gegenwart eine Säureakzeptors durchgeführt. Es kommen hierbei vorzugsweise diejenigen Säurebindemittel in Betracht, die oben bei der Beschreibung des erfindungsgemäßen Verfahrens (a) angegeben wurden.

Das erfindungsgemäße Verfahren (f) wird vorzugsweise unter Verwendung eines Verdünnungsmittels durchgeführt. Es kommen hierbei vorzugsweise diejenigen Verdünnungsmittel in Frage, die oben bei der Beschreibung des erfindungsgemäßen Verfahrens (a) angegeben wurden.

Die Reaktionstemperaturen können bei dem erfindungsgemäßen Verfahren (f) in einem größeren Bereich variiert werden. Im allgemeinen arbeitet man bei Temperaturen zwischen 0°C und 150°C, vorzugsweise bei Temperaturen zwischen 20°C und 100°C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (f) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (f) jeweils nach üblichen Methoden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhiopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich durch hervorragende insektizide Wirksamkeit aus. Sie zeigen sowohl als Blattinsektizide als auch als Bodeninsektizide sehr starke Wirkung, z.B. gegen Käferlarven (z.B. Phaedon cocheariae), gegen Reiszikaden (z.B. Nephotettix cincticeps) sowie gegen Blattläuse (z.B. Myzus persicae und Aphis fabae).

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sog. Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaltlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie z.B. durch Injektionen (intramuskulär, durch nasale Applikation, durch dermale Anwendung in Form beispielsweise des Tauchens oder Badens (Dippen), Sprühens (Spray), Aufgießens Pour-on und Spot-on), des Waschens, des Einpuderns sowie mit Hilfe von wirkstolthaltigen Formkörpern, wie Halsbändern, Ohrmarken, Schwanzmarken, Gliedmaßenbändern, Halftern, Markierungsvorrichtungen usw.

Die Wirkstoffe können in Abhängigkeit von ihren jeweiligen phydikalischen und/oder chemischen Eichenschatten in üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen. -spiralen u.ä., sowie ULV-Kalt- und Warmnebel-Formulieiungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt, der aus den handelsüblichen Formulierungen bereiteten Anwendungsformen, kann in weiten Bereichen variieren. Die Wirkstoftkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

### Herstellungsbeispiele:

### Beispiel 1

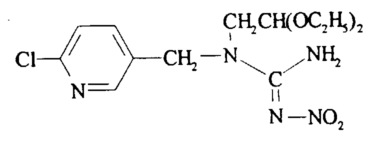

### <Verfahren (a)>

Eine Mischung aus 1,15 g (5,0 mMol) 1-(6-Chlor-pyridin-3-yl-methyl)-2-nitroguanidin, 0,76 g (5,5 mMol) Kaliumcarbonat, 0,09 g (0,5 mMol) Cäsiumchlorid, 0,92 ml (5,5 mMol) Bromacetaldehyd-diethylacetal und 5 ml Dimethylformamid wird unter Rühren auf 125°C erhizt und zwei Stunden bei dieser Temperatur gerührt. Nach Zugabe von weiteren 0,76 g (5,5 mMol) Kaliumcarbonat und weiteren 0,92 ml (5,5 mMol) Bromacetaldehyd-diethylacetal wird das Gemisch weitere zwei Stunden bei 125°C gerührt.

Dann wird nach Abkühlen auf Raumtemperatur mit Eiswasser auf etwa das vierfache Volumen verdünnt und mit Methylenchlorid extrahiert. Die organische Phase wird mit Natriumsulfat getrocknet und filtriert. Das Filtrat wird eingeengt und der Rückstand mit eiskaltem Cyclohexan digeriert. Das dabei ungelöst gebliebene sirupöse Rohprodukt wird durch Säulenchromatographie (Kieselgel; Methylenchlorid | Essigsäureethylester 1:1) gereinigt.
Man erhält so 0,16 g (9% der Theorie) 1-(6-Chlor-pyridin-3-yl-methyl) -1-(2,2-diethoxyethyl)-2-nitro-guanidin als amorphe 2.Fraktion.

| | | |
|---|---|---|
| ¹H-NMR (CDCl₃, δ): | -CH (OC₂H₅)₂ | 4.59 ppm (Triplett) |
| | -CH₂-CH (OC₂H₅)₂ | 3.39 ppm (Duplett) |

### Beispiel 2

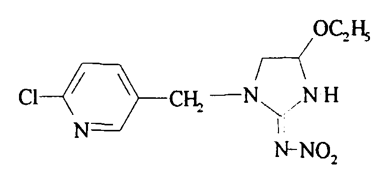

### <Verfahren (b)>

Eine Mischung von 1,45 g (4,2 mMol) 1-(6-Chlor-pyridin-3-yl-methyl)-1-(2,2-diethoxy-ethyl)-2-nitro-guanidin und 44 ml Ethanol wird auf 78°C erhitzt und nach Zugabe von 0,725 ml (7,25 mMol) konzentrierter Salzsäure 15 Minuten bei dieser Temperatur gerührt.
Nach Abkühlen auf Raumtemperatur wird mit Essigsäureethylester auf etwa das dreifache Volumen verdünnt und mit wässriger Kaliumhydrogencarbonatlösung neutralisiert. Die organische Phase wird abgetrennt, mit Natriumsulfat getrocknet und filtriert. Das Filtrat wird eingeengt und der Rückstand durch Säulenchromatographie (Kieselgel; Methylenchlorid | Methanol 15:1) gereinigt.
Man erhält 1,2 g (95% der Theorie) 1-(6-Chlor-pyridin-3-yl-methyl)-2-nitroimino-4-ethoxy-imidazolidin als amorphe 2. Fraktion.

| | | |
|---|---|---|
| ¹H-NMR (DMSO₃, δ): | 4-CH | 5.90 ppm |
| | 5-CH₂ | 3.70 ppm (Multiplett) |

### Beispiel 3

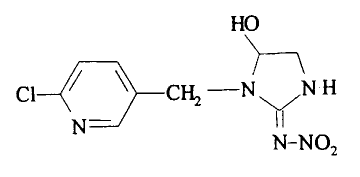

### 〈Verfahren (c)〉

4,2 g (15,5 mMol) 1-(6-Chlor-pyridin-3-yl-methyl)-2-nitroimino-5-oxo-imidazolidin werden in 495 ml Tetrahydrofuran gelöst und dann werden 25,2 ml (25,2 mMol HCl) 1N-Salzsäure dazugegeben.
Das Gemisch wird auf 15°C gekühlt und bei dieser Temperatur werden 42 ml einer 4%igen wässrigen Lösung von Natriumborhydrid tropfenweise dazugegeben. Nach 30-minütigem Nachrühren wird vom ungelösten Feststoff abdekantiert und die Lösung wird eingeengt. Dann wird mit 200 ml gesättigter wässriger Natriumchloridlösung versetzt und dreimal mit je 200 ml Essigsäureethylester
extrahiert. Die vereinigten organischen Phasen werden mit Natriumsulfat getrocknet und filtriert. Das Filtrat wird eingeengt, der Rückstand mit 200 ml Essigsäureethylester | Acetonitril (10:1) digeriert und das ungelöst gebliebene Produkt durch Absaugen isoliert.
- 1. Produktfraktion :: 0,6 g braungelbe Kristalle. Das Filtrat wird über eine Kieselgelsäule fraktioniert.
- 2. Produktfraktion :: 2,8 g farblose Kristalle;
- Schmelzpunkt :: 192°C
- Gesamtausbeute :: 3,4 g (80% der Theorie) 1-(6-Chlor-pyridin-3-yl-methyl)-2-nitroimino-5-hydroxy-imidazolidin.

### Beispiel 4

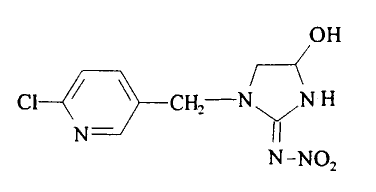

### <Verfahren (d)>

Eine Mischung aus 0,36 g (1,2 mMol) 1-(6-Chlor-pyridin 3-yl-methyl)-2-nitroimino-4-ethoxy-imidazolidin, 36 ml Tetrahydrofuran, 36 ml gesättigter wässriger Natriumchloridlösung und 2,4 ml 1N-Salzsäure wird 105 Minuten bei 45°C gerührt.
Die zweiphasige Reaktionsmischung wird nach Abkühlen auf Raumtemperatur mit wässriger Kaliumhydrogencarbonatlösung auf pH 8 eingestellt und dann dreimal mit Essigsäureethylester extrahiert. die vereinigten Extrakte werden mit Natriumsulfat getrocknet und filtriert. Das Filtrat wird eingeengt, der Rückstand mit Diethylether digeriert und das kristallin angefallene Produkt durch Absaugen isoliert.
Man erhält 0,143 g (44% der Theorie) 1-(6-Chlor-pyridin-3-yl-methyl)-2-nitroimino-4-hydroxy-imidazolidin vom Schmelzpunkt 170°C.

### Beispiel 5

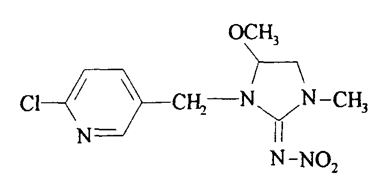

### <Verfahren (e)>

1,1 g (4,0 mMol) 1-(6-Chlor-pyridin-3-yl-methyl)-2-nitroimino-5-hydroxy-imidazolidin werden in 10 ml Dimethylformamid gelöst und dann werden 0,24 g (8,0 mMol) Natriumhydrid portionsweise unter Rühren dazugegeben. Das Gemisch wird 30 Minuten bei 15°C gerührt. Dann werden bei +5°C 0,5 ml (8,0 mMol) Methyliodid zugetropft und das Reaktionsgemisch wird 2 Stunden bei 20°C gerührt. Anschließend wird mit Wasser auf etwa das dreifache Volumen verdünnt und dreimal mit Essigsäureethylester extrahiert. Die vereinigten Extrakte werden mit Natriumsulfat getrocknet und filtriert. Das Filtrat wird eingeengt und der Rückstand säulenchromatographisch (Kieselgel | Essigsäureethylester) fraktioniert.
Man erhält 0,80 g (67% der Theorie) 1-(6-Chlor-pyridin-3-yl-methyl)-2-nitroimino-3-methyl-5-methoxy-imidazolidin als amorphe 3.Fraktion.

| | | |
|---|---|---|
| ¹H-NMR(CDCl₃,) : | OCH₃ | 3.26 ppm (Singulett) |
| | NCH₃ | 3.01 ppm (Singulett) |

Analog zu den Herstellungsbeispielen 1 bis 5 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

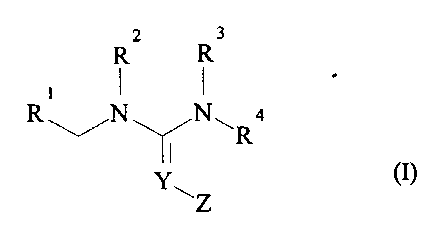

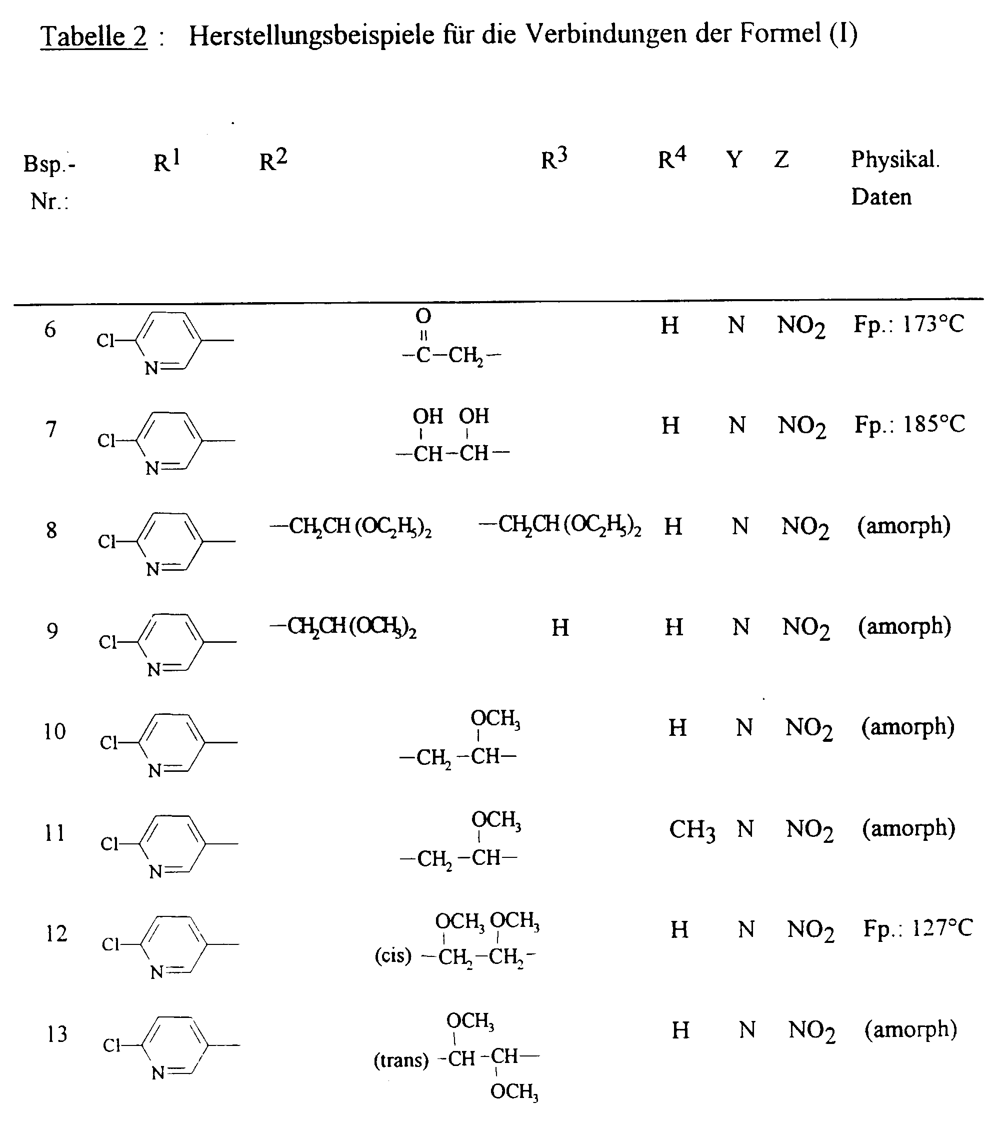

### Anwendungsbeispiele

### Beispiel A

### Phaedon-Larven-Test

- Lösungsmittel :: 7 Gewichtsteile Dimethylformamid
- Emulgator :: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die gemäß den Herstellungsbeispielen (1), (3), (5) und (6) erhaltenen Verbindungen bei einer Wirkstoffkonzentration von 0,1 % eine 100 %ige Abtötung der Versuchstiere nach 7 Tagen.

### Beispiel B

### Nephotettix-Test

- Lösungsmittel :: 7 Gewichtsteile Dimethylformamid
- Emulgator :: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Testtiere abgetötet wurden; 0 % bedeutet, daß keine Testtiere abgetötet wurden.

Bei diesem Test zeigen z.B. die gemäß den Herstellungsbeispielen (1), (3), (5) und (8) erhaltenen Verbindungen bei einer Wirkstoffkonzentration von 0,1 % eine 100 %ige Abtötung der Testtiere nach 6 Tagen.

### Beispiel C

### Myzus-Test

- Lösungsmittel :: 7 Gewichtsteile Dimethylformamid
- Emulgator :: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandel.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die gemäß den Herstellungsbeispielen (1) und (5) erhaltenen Verbindungen bei einer Wirkstoffkonzentration von 0,1 % eine 100 %ige Abtötung der Testtiere nach 6 Tagen.

### Beispiel D

### Aphis-Test (systemische Wirkung)

- Lösungsmittel :: 7 Gewichtsteile Dimethylformamid
- Emulgator :: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Testtiere abgetötet wurden; 0 % bedeutet, daß keine Testtiere abgetötet wurden.

Bei diesem Test zeigen z.B. die gemäß den Herstellungsbeispielen (3) und (5) erhaltenen Verbindungen bei einer Wirkstoffkonzentration von 0,1 % eine 100 %ige Abtötung der Testtiere nach 4 Tagen.

### Beispiel E

### Grenzkonzentrations-Test / Wurzelsystemische Wirkung

- Testinsekt :: Myzus persicae
- Lösungsmittel :: 4 Gewichtsanteile Aceton
- Emulgator :: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt.
Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet.
Sie ist 100 %ig, wenn alle Testtiere abgetötet sind und 0 %ig, wenn noch genau so viele Testinsekten leben wie bei der unbehandelten Kontrolle.

Bei diesem Test zeigen z.B. die gemäß Herstellungsbeispiel (1) und (3) erhaltenen Verbindungen bei einer Wirkstoffkonzentration von 20 ppm eine 100 %ige Abtötung der Testtiere.

### Beispiel F

### Blowfly-Larven-Test

- Testtiere :: Lucilia cuprina-Larven
- Emulgator :: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration. Etwa 20 Lucilia cuprina res.-Larven werden in ein Teströhrchen gebracht, welches ca.

1 cm³ Pferdefleisch und 0,5 ml der Wirkstoffzubereitung enthält. Nach 24 Stunden wird die Wirksamkeit der Wirkstoffzubereitung ermittelt. Dabei bedeutet 100 %, daß alle Blowfly-Larven abgetötet wurden; 0 % bedeutet, daß keine Blowfly-Larven abgetötet wurden.

Bei diesem Test zeigen z.B. die Verbindungen gemäß den Herstellungsbeispielen (1), (2), (4), (5) und (8) gute Wirksamkeit.

## Patentansprüche

1. Substituierte Aza(cyclo)alkane der allgemeinen Formel (I) 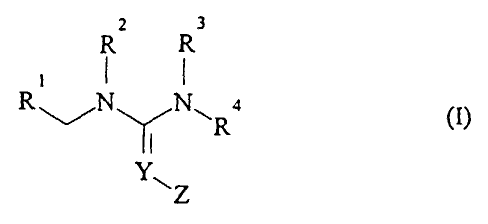 in welcher
R¹ für eine fünf- bis sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl, welche durch Fluor,Chlor,Brom, Iod,Cyano,Nitro, C₁-C₄-Alkyl (welches gegebenenfalls durch Fluor und/oder Chlor substituiert ist), C₂-C₄-Alkenyl (welches gegebenenfalls durch Fluor und/oder Chlor substituiert ist), C₂-C₄-Alkinyl, C₁-C₄-Alkoxy (welches gegebenenfalls durch Fluor und/oder Chlor substituiert ist), C₃-C₄-Alkenyloxy (welches gegebenenfalls durch Fluor und/oder Chlor substituiert ist), C₃-C₄-Alkinyloxy, C₃-C₄-Alkylthio (welches gegebenenfalls durch Fluor und/oder Chlor substituiert ist), C₃-C₄-Alkenylthio (welches gegebenenfalls durch Fluor und/oder Chlor substituiert ist), C₃-C₄-Alkinylthio, C₁ C₄-Alkylsulfinyl (welches gegebenenfalls durch Fluor und/oder Chlor substituiert ist), C₁-C₄-Alkylsulfonyl (welches gegenenenfalls durch Fluor und /oder Chlor substituiert ist), Amino, C₁-C₄-Alkylamino, Di-(C₁-C₄-alkyl)-amino, Phenyl, Phenoxy, Phenylthio, Phenylamino, Benzyl, Formylamino, C₁-C₄-Alkyl-carbonylamino, Formyl, Carbamoyl, C₁-C₄-Alkylcarbonyl und/oder C₁-C₄-Alkoxy-carbonyl substituiert ist, steht
R für C₁-C₆-Hydroxyalkyl, Dihydroxy-C₁-C₆-alkyl, C₁-C₄-Alkoxy-C₁-C₄-alkyl, Di-(C₁-C₄-alkoxy)-C₁-C₄-alkyl steht,
R³ für Wasserstoff, C₁-C₆-Alkyl, C₁-C₆-Hydroxyalkyl, Dihydroxy-C₁-C₆-alkyl, C₁-C₄-Alkoxy-C₁-C₄-alkyl, Di-(C₁-C₄-alkoxy)-C₁-C₄-alkyl oder
R und R³ zusammen für Hydroxy-C₂-C₄-alkandiyl, Dihydroxy-C₂-C₄-alkandiyl, C₁-C₄-Alkoxy-C₂-C₄-alkandiyl, Di-(C₁-C₄-alkoxy)-C₂-C₄-alkandiyl, Oxo-C₂-C₄-alkandiyl oder Dioxo-C₂-C₄-alkandiyl stehen,
R⁴ für Wasserstoff, C₁-C₄-Alkyl (welches gegebenenfalls durch Fluor, Chlor, Cyano, C₁-C₄-Alkoxy, C₁-C₄-Alkylthio, Di-(C₁-C₄-alkyl)-amino, Trimethylsilyl, C₁-C₄-Alkoxy-carbonyl, Carboxy, Carbamoyl, C₁-C₄-Alkyl-aminocarbonyl, Di-(C₁-C₃-alkyl)-aminocarbonyl, oder durch eine heterocyclische Gruppierung, wie sie oben für R¹ (einschließlich der möglichen Substituenten) vorzugsweise definiert ist, substituiert ist), C₂-C₄-Alkenyl (welches gegebenenfalls durch Fluor oder Chlor substituiert ist), C₂-C₄-Alkinyl, Benzyl (welches gegebenenfalls durch Fluor, Chlor, Cyano, Nitro, C₁-C₂-Alkyl, Trifluormethyl, C₁-C₂-Alkoxy oder C₁-C₂-Alkoxy-carbonyl substituiert ist), Formyl, C₁-C₂₀-Alkyl-carbonyl (welches gegebenenfalls durch Fluor, Chlor, Brom, Phenyl, Phenoxy oder C₁-C₄-Alkoxy substituiert ist), C₃-C₆-Cycloalkylcarbonyl (welches gegebenenfalls durch Fluor, Chlor und/oder C₁-C₄-Alkyl substituiert ist), C₂-C₂₀-Alkenyl-carbonyl (welches gegebenenfalls durch Fluor und/oder Chlor substituiert ist), Phenylcarbonyl oderNaphthylcarbonyl (welche gegebenenfalls durch Fluor, Chlor, Brom, C₁-C₄-Alkyl, Trifluormethyl, Cyano, Nitro, C₁-C₄-Alkoxy und/oder C₁-C₄-Alkoxy-carbonyl substituiert sind), C₁-C₂₀-Alkoxy-carbonyl, Benzyloxy-carbonyl, Phenoxycarbonyl, C₁-C₄-Alkylthiocarbonyl, Benzylthio-carbonyl, Phenylthio-carbonyl, C₁-C₆-Alkylamino-carbonyl, Di-(C₁-C₄-alkyl)-amino-carbonyl, Phenylamino-carbonyl (welches gegebenenfalls durch Fluor, Chlor, Brom, Cyano, Nitro, C₁-C₄-Alkyl, Trifluormethyl, C₁-C₄-Alkoxy, C₁-C₂-Fluoralkoxy, C₁-C₂-Chlor-fluoralkoxy, C₁-C₄-Alkylthio, C₁-C₂-Fluoralkylthio, C₁-C₂-Chlorfluoralkylthio oder C₁-C₄-Alkoxycarbonyl substituiert ist), Benzoylamino-carbonyl (welches gegebenenfalls durch Fluor, Chlor, Brom, Methyl oder Trifluormethyl substituiert ist), Phenylsulfonylamino-carbonyl (welches gegebenenfalls durch Fluor, Chlor, Brom, Methyl, Trifluormethyl, C₁-C₄-Alkoxy, C₁-C₂-Fluoralkoxy, C₁-C₂-Chlorfluoralkoxy oder C₁-C₄-Alkoxy-carbonyl substituiert ist), C₁-C₄-Alkylthio (welches gegebenenfalls durch Fluor und/oder Chlor substituiert ist), Phenylthio (welches gegebenenfalls durch Fluor, Chlor, Brom, Nitro oder Methyl substituiert ist), C₁-C₄-Alkylsulfinyl, C₁-C₄-Alkylsulfonyl (welches gegebenenfalls durch Fluor und/oder Chlor substituiert ist), Phenylsulfinyl (welches gegebenenfalls durch Fluor, Chlor, Brom, Nitro oder Methyl substituiert ist), Phenylsulfonyl oder Naphthylsulfonyl (welche gegebenenfalls durch Fluor, Chlor, Brom, Cyano, Nitro, Methyl, Trifluormethyl, C₁-C₄-Alkoxy, C₁-C₂-Fluoralkoxy, C₁-C₂-Chlorfluoralkoxy und/oder C₁-C₄-Alkoxy-carbonyl substituiert sind), Dimethyl(thio)phosphoryl, C₁-C₄-Alkyl-C₁-C₄-alkoxy-(thio)phosphoryl oder Di(C₁-C₄-alkoxy)-(thio)phosphoryl steht,
Y für Stickstoff oder eine CH-Gruppierung steht und
Z für Cyano oder Nitro steht.

2. Verfahren zur Herstellung von substituierten Aza(cyclo)alkanen der allgemeinen Formel (I) gemäß Anspruch 1 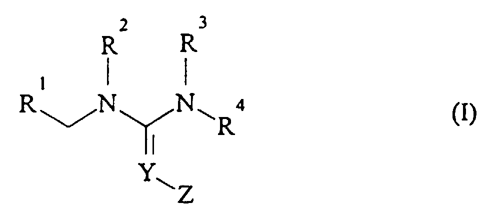 in welcher
R¹ für eine fünf- bis sechsgliedrige heterocyclische Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl, welche durch Fluor,Chlor,Brom,Iod,Cyano,Nitro, C₁-C₄-Alkyl (welches gegebenenfalls durch Fluor und/oder Chlor substituiert ist), C₂-C₄-Alkenyl (welches gegebenenfalls durch Fluor und/oder Chlor substituiert ist), C₂-C₄-Alkinyl, C₁-C₄-Alkoxy (welches gegebenenfalls durch Fluor und/oder Chlor substituiert ist), C₃-C₄-Alkenyloxy (welches gegebenenfalls durch Fluor und/oder Chlor substituiert ist), C₃-C₄-Alkinyloxy, C₃-C₄-Alkylthio (welches gegebenenfalls durch Fluor und/oder Chlor substituiert ist), C₃-C₄-Alkenylthio (welches gegebenenfalls durch Fluor und/oder Chlor substituiert ist), C₃-C₄-Alkinylthio, C₁C₄-Alkylsulfinyl (welches gegebenenfalls durch Fluor und/oder Chlor substituiert ist), C₁-C₄-Alkylsulfonyl (welches gegenenenfalls durch Fluor und /oder Chlor substituiert ist), Amino, C₁-C₄-Alkylamino, Di-(C₁-C₄-alkyl)-amino, Phenyl, Phenoxy, Phenylthio, Phenylamino, Benzyl, Formylamino, C₁-C₄-Alkyl-carbonylamino, Formyl, Carbamoyl, C₁-C₄-Alkylcarbonyl und/oder C₁-C₄-Alkoxy-carbonyl substituiert ist, steht
dadurch gekennzeichnet, daß man
(a) Azaalkane der allgemeinen Formel (II) 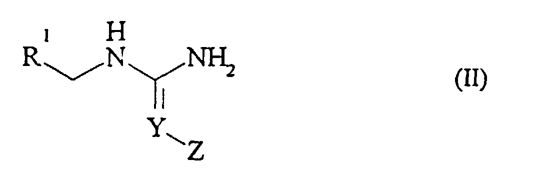 in welcher
R¹, Y und Z die oben angegebene Bedeutung haben,
mit Halogenverbindungen der allgemeinen Formel (IIIa)
X-R (IIIa)
und/oder Halogenverbindungen der allgemeinen Formel (IIIb)
X-R³ (IIIb)
worin
R und R³ die oben angegebene Bedeutung haben und
X für Halogen steht,
gegebenenfalls in Gegenwart eines Säureakzeptors, gegebenenfalls in Gegenwart eines Katalysators oder gegebenenfalls in Gegenwart eines Verdünnungsmittels umsetzt,
oder daß man
(b) Verbindungen der allgemeinen Formel (1), in welcher R oder R³ für Dialkoxyalkyl steht und R¹, R⁴, Y und Z die oben angegebene Bedeutung haben, gegebenenfalls in Gegenwart eines Reaktionshilfsmittels und gegebenenfalls in Gegenwart eines Verdünnungsmittels erhitzt,
oder daß man
(c) Verbindungen der allgemeinen Formel (I), in welcher R und R³ zusammen für Oxoalkandiyl stehen und R¹, R⁴,Y und Z die oben angegebene Bedeutung haben,
mit einem Hydrierungsmittel, gegebenenfalls in Gegenwart eines Reaktionshilfsmittels und gegebenenfalls in Gegenwart eines Verdünnungsmittels umsetzt,
oder daß man
(d) Verbindungen der allgemeinen Formel (I), in welcher R und R³ zusammen für Alkoxyalkandiyl oder Dialkoxyalkandiyl stehen und R¹, R⁴, Y und Z die oben angegebene Bedeutung haben,
mit einem Hydrogenhalogenid und/oder einem Alkalimetallhalogenid sowie in Gegenwart von Verdünnungsmitteln umsetzt,
oder daß man
(e) Verbindungen der allgemeinen Formel (I), in welcher R und R³ zusammen für Hydroxyalkandiyl oder Dihydroxyalkandiyl stehen und R¹, R⁴, Y und Z die oben angegebene Bedeutung haben,
mit einem Alkylierungsmittel der allgemeinen Formel (IV)
X¹-R (IV)
in welcher
R für Alkyl steht und
X¹ für Halogen oder die Gruppierung -O-SO₂-O-R steht, worin R die oben angegebene Bedeutung hat,
gegebenenfalls in Gegenwart eines Säurebindemittels und gegebenenfalls in Gegenwart eines Verdünnungsmittels umsetzt,
oder daß man
(f) Verbindungen der allgemeinen Formel (1), in welcher R⁴ für Wasserstoff steht und R¹, R, R³, Y und Z die oben angegebene Beutung haben,
mit Halogenverbindungen der allgemeinen Formel (V)
X-R⁴ (V)
in welcher
X für Halogen steht und
R⁴ mit Ausnahme von Wasserstoff die oben angegebene Bedeutung hat,
gegebenenfalls in Gegenwart eines Säureakzeptors und gegebenenfalls in Gegenwart eines Verdünnungsmittels umsetzt.

3. Substituierte Aza(cyclo)alkane der Formel (I) gemäß Anspruch 1, in welcher
R¹ für eine fünf- bzw. sechsgliedrige heterocyclische Gruppierung aus der Reihe Pyrazoyl, 1,2,4-Triazolyl, Oxazolyl, Isoxazolyl, Thiazolyl, Isothiazolyl, 1,2,5-Thiadiazolyl, Pyridyl, Pyrazinyl und Pyrimidinyl steht, welche durch Fluor, Chlor, Brom, Cyano, Nitro, C₁-C₂-Alkyl (welches gegebenenfalls durch Fluor und/oder Chlor substituiert ist), C₁-C₂-Alkoxy (welches gegebenenfalls durch Fluor und/oder Chlor substituiert ist), C₁-C₂-Alkylthio (welches gegebenenfalls durch Fluor und/oder Chlor substituiert ist) oder C₁-C₂-Alkylsulfonyl (welches gegebenenfalls durch Fluor und/oder Chlor substituiert ist) substituiert ist,
R für C₁-C₄-Hydroxyalkyl, Dihydroxy-C₁-C₄-alkyl, C₁-C₃-Alkoxy-C₁-C₃-alkyl, Di-(C₁-C₃-alkoxy)-C₁-C₃-alkyl,
R³ für Wasserstoff, C₁-C₄-Alkyl, C₁-C₄-Hydroxyalkyl, Dihydroxy-C₁-C₄-alkyl, C₁-C₃-Alkoxy-C₁-C₃ -alkyl, Di-(C₁-C₃-alkoxy)-C₁-C₃-alkyl oder
R und R³ zusammen für Hydroxy-C₂-C₃-alkandiyl, Dihydroxy-C₂-C₃-alkandiyl, C₁-C₃-Alkoxy-C₂-C₃-alkandiyl, Di(C₁-C₃-alkoxy)-C₂-C₃-alkandiyl, Oxo-C₂-C₃-alkandiyl oder Dioxo-C₂-C₃-alkandiyl stehen,
R⁴ für Wasserstoff, Methyl, Ethyl, Allyl, Propargyl, Formyl, C₁-C₈-Alkylcarbonyl (welches gegebenenfalls durch Fluor und/oder Chlor substituiert ist), C₁-C₈-Alkoxy-carbonyl, Benzyloxy-carbonyl, Phenoxycarbonyl, Benzyl (welches gegebenenfalls durch Fluor oder Chlor substituiert ist) oder Di-(C₁-C₂-alkoxy)-(thio)phosphoryl steht,
Y für Stickstoffoder eine CH-Gruppierung steht und
Z für Cyano oder Nitro steht.

4. Substituierte Aza(cyclo)alkane der Formel (I) gemäß Anspruch 1, in welcher
R¹ für 6-Chlor-3-pyridyl (6-Chlor-pyridin-3-yl) oder für 2-Chlor-5-thiazolyl (2-Chlor-thiazol-5-yl) steht,
R für Hydroxymethyl, Hydroxyethyl, Hydroxypropyl, Dihydroxyethyl, Dihydroxypropyl, Methoxymethyl, Ethoxymethyl, Methoxyethyl, Ethoxyethyl, Dimethoxyethyl, Diethoxyethyl,
R³ für Wasserstoff, Methyl, Ethyl, Hydroxymethyl, Hydroxyethyl, Hydroxypropyl, Dihydroxyethyl, Dihydroxypropyl, Methoxymethyl, Ethoxymethyl, Methoxyethyl, Ethoxyethyl, Dimethoxyethyl, Diethoxyethyl,
R und R³ zusammen für Oxoethan-1,2-diyl, Dioxoethan-1,2-diyl, Hydroxyethan-1,2-diyl, Dihydroxyethan-1,2-diyl, 1,2-diyl, Methoxyethan- 1,2-diyl, Ethoxyethan-1,2-diyl, Dimethoxyethan-1,2-diyl oder Diethoxyethan-1,2-diyl stehen,
R⁴ für Wasserstoff oder Methyl steht,
Y für Stickstoff oder eine CH-Gruppierung steht und
Z für Cyano oder Nitro steht.

5. Schädlingsbekämpfüngsmittel, gekennzeichnet durch einen Gehalt an mindestens einem substituierten Aza(cyclo)alkan der Formel (I) gemäß Anspruch 1.

6. Verwendung von substituierten Aza(cyclo)alkanen der Formel (I) gemäß Anspruch 1 zur Bekämpfüng von Schädlingen.

7. Verfahren zur Bekämpfung von Schädlingen, dadurch gekennzeichnet, daß man substituierten Aza(cyclo)alkanen der Formel (I) gemäß Anspruch 1 auf Schädlinge und/oder ihren Lebensraum einwirken läßt.

8. Verfahren zur Herstellung von Schädlingsbekämpfüngsmitteln, dadurch gekennzeichnet, daß man substituierte Aza(cyclo)alkane der Formel (I) gemäß Anspruch 1 mit Streckmitteln und/oder oberflächenaktiven Mitteln vermischt.

## Claims

1. Substituted aza(cyclo)alkanes of the general formula (I) 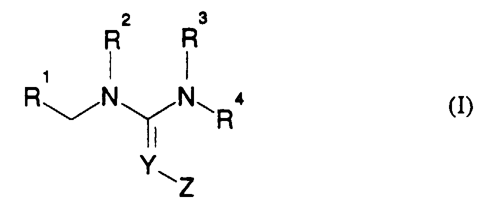 in which
R¹ represents a five- to six-membered heterocyclic grouping from the series comprising furyl, thienyl, pyrrolyl, pyrazolyl, imidazolyl, 1,2,3-or 1,2,4-triazolyl, oxazolyl, isoxazolyl, 1,2,4-or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, which is substituted by fluorine, chlorine, bromine, iodine, cyano, nitro, C₁-C₄-alkyl (which is optionally substituted by fluorine and/or chlorine), C₂-C₄-alkenyl (which is optionally substituted by fluorine and/or chlorine), C₂-C₄-alkinyl, C₁-C₄-alkoxy (which is optionally substituted by fluorine and/or chlorine), C₃-C₄-alkenyloxy (which is optionally substituted by fluorine and/or chlorine), C₃-C₄-alkinyloxy, C₃-C₄-alkylthio (which is optionally substituted by fluorine and/or chlorine), C₃-C₄-alkenylthio (which is optionally substituted by fluorine and/or chlorine), C₃-C₄-alkinylthio, C₁-C₄-alkylsulphinyl (which is optionally substituted by fluorine and/or chlorine), C₁-C₄-alkylsulphonyl (which is optionally substituted by fluorine and/or chlorine), amino, C₁-C₄-alkylamino, di-(C₁-C₄-alkyl)-amino, phenyl, phenoxy, phenylthio, phenylamino, benzyl, formylamino, C₁-C₄-alkyl-carbonylamino, formyl, carbamoyl, C₁-C₄-alkylcarbonyl and/or C₁-C₄-alkoxy-carbonyl,
R represents C₁-C₆-hydroxyalkyl, dihydroxy-C₁-C₆-alkyl, C₁-C₄-alkoxy-C₁-C₄-alkyl or di-(C₁-C₄-alkoxy)-C₁-C₄-alkyl,
R³ represents hydrogen, C₁-C₆-alkyl, C₁-C₆-hydroxyalkyl, dihydroxy-C₁-C₆-alkyl, C₁-C₄-alkoxy-C₁-C₄-alkyl or di-(C₁-C₄-alkoxy)-C₁-C₄-alkyl, or
R and R³ together represent hydroxy-C₂-C₄-alkanediyl, dihydroxy-C₂-C₄-alkanediyl, C₁-C₄-alkoxy-C₂-C₄-alkanediyl, di-(C₁-C₄-alkoxy)-C₂-C₄-alkanediyl, oxo-C₂-C₄-alkanediyl or dioxo-C₂-C₄-alkanediyl,
R⁴ represents hydrogen, C₁-C₄-alkyl (which is optionally substituted by fluorine, chlorine, cyano, C₁-C₄-alkoxy, C₁-C₄-alkylthio, di-(C₁-C₄-alkyl)-amino, trimethylsilyl, C₁-C₄-alkoxycarbonyl, carboxyl, carbamoyl, C₁-C₄-alkyl-aminocarbonyl, di-(C₁-C₃-alkyl) -aminocarbonyl, or by a heterocyclic grouping as is preferably defined above for R¹ (including the possible substituents)), C₂-C₄-alkenyl (which is optionally substituted by fluorine or chlorine), C₂-C₄-alkinyl, benzyl (which is optionally substituted by fluorine, chlorine, cyano, nitro, C₁-C₂-alkyl, trifluoromethyl, C₁-C₂-alkoxy or C₁-C₂-alkoxycarbonyl), formyl, C₁-C₂₀-alkyl-carbonyl (which is optionally substituted by fluorine, chlorine, bromine, phenyl, phenoxy or C₁-C₄-alkoxy), C₃-C₆-cycloalkylcarbonyl (which is optionally substituted by fluorine, chlorine and/or C₁-C₄-alkyl), C₂-C₂₀-alkenyl-carbonyl (which is optionally substituted by fluorine and/or chlorine), phenylcarbonyl or naphthylcarbonyl (which are optionally substituted by fluorine, chlorine, bromine, C₁-C₄-alkyl, trifluoromethyl, cyano, nitro, C₁-C₄-alkoxy and/or C₁-C₄-alkoxycarbonyl), C₁-C₂₀-alkoxy-carbonyl, benzyloxycarbonyl, phenoxycarbonyl, C₁-C₄-alkylthiocarbonyl, benzylthio-carbonyl, phenylthio-carbonyl, C₁-C₆-alkylamino-carbonyl, di-(C₁-C₄-alkyl)-amino-carbonyl, phenylaminocarbonyl (which is optionally substituted by fluorine, chlorine, bromine, cyano, nitro, C₁-C₄-alkyl, trifluoromethyl, C₁-C₄-alkoxy, C₁-C₂-fluoroalkoxy, C₁-C₂-chloro-fluoroalkoxy, C₁-C₄-alkylthio, C₁-C₂-fluoroalkylthio, C₁-C₂-chlorofluoroalkylthio or C₁-C₄-alkoxycarbonyl), benzoylamino-carbonyl (which is optionally substituted by fluorine, chlorine, bromine, methyl or trifluoromethyl), phenylsulphonylaminocarbonyl (which is optionally substituted by fluorine, chlorine, bromine, methyl, trifluoromethyl, C₁-C₄-alkoxy, C₁-C₂-fluoroalkoxy, C₁-C₂-chlorofluoroalkoxy or C₁-C₄-alkoxy-carbonyl), C₁-C₄-alkylthio (which is optionally substituted by fluorine and/or chlorine), phenylthio (which is optionally substituted by fluorine, chlorine, bromine, nitro or methyl), C₁-C₄-alkylsulphinyl, C₁-C₄-alkylsulphonyl (which is optionally substituted by fluorine and/or chlorine), phenyl/ sulphinyl (which is optionally substituted by fluorine, chlorine, bromine, nitro or methyl), phenylsulphonyl or naphthylsulphonyl (which are optionally substituted by fluorine, chlorine, bromine, cyano, nitro, methyl, trifluoromethyl, C₁₋C₄-alkoxy, C₁-C₂-fluoroalkoxy, C₁-C₂-chlorofluoroalkoxy and/or C₁-C₄-alkoxy-carbonyl), dimethyl(thio)phosphoryl, C₁-C₄-alkyl-C₁-C₄-alkoxy-(thio)phosphoryl or di(C₁-C₄-alkoxy)-(thio)phosphoryl,
Y represents nitrogen or a CH grouping and
Z represents cyano or nitro.

2. Process for the preparation of substituted aza(cyclo)alkanes of the general formula (I) 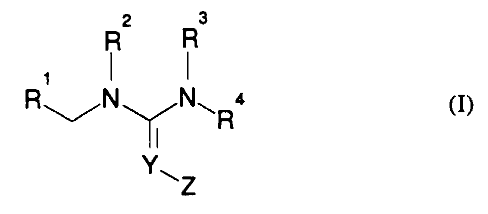 in which
R¹ represents a five- to six-membered heterocyclic grouping from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, which is substituted by fluorine, chlorine, bromine, iodine, cyano, nitro, C₁-C₄-alkyl (which is optionally substituted by fluorine and/or chlorine), C₂-C₄-alkenyl (which is optionally substituted by fluorine and/or chlorine), C₂-C₄-alkinyl, C₁-C₄-alkoxy (which is optionally substituted by fluorine and/or chlorine), C₃-C₄-alkenyloxy (which is optionally substituted by fluorine and/or chlorine), C₃-C₄-alkinyloxy, C₃-C₄-alkylthio (which is optionally substituted by fluorine and/or chlorine), C₃-C₄-alkenylthio (which is optionally substituted by fluorine and/or chlorine), C₃-C₄-alkinylthio, C₁-C₄-alkylsulphinyl (which is optionally substituted by fluorine and/or chlorine), C₁-C₄-alkylsulphonyl (which is optionally substituted by fluorine and/or chlorine), amino, C₁-C₄-alkylamino, di-(C₁-C₄-alkyl)-amino, phenyl, phenoxy, phenylthio, phenylamino, benzyl, formylamino, C₁-C₄-alkyl-carbonylamino, formyl, carbamoyl, C₁-C₄-alkylcarbonyl and/or C₁-C₄-alkoxy-carbonyl,
characterised in that
(a) azaalkanes of the general formula (II) 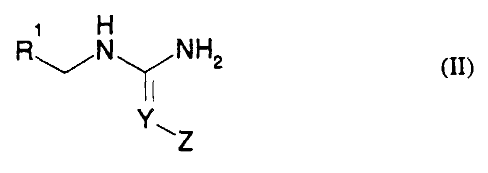 in which
R¹, Y and Z have the abovementioned meaning,
are reacted with halogen compounds of the general formula (IIIa)
X-R (IIIa)
and/or halogen compounds of the general formula (IIIb)
X-R³ (IIIb)
in which
R and R³ have the abovementioned meaning and
X represents halogen,
optionally in the presence of an acid acceptor, optionally in the presence of a catalyst or optionally in the presence of a diluent,
or in that
(b) compounds of the general formula (I), in which R or R³ represents dialkoxyalkyl and R¹, R⁴, Y and Z have the abovementioned meaning,
are heated, optionally in the presence of a reaction aid and optionally in the presence of a diluent,
or in that
(c) compounds of the general formula (I), in which R and R³ together represent oxoalkanediyl and R¹, R⁴, Y and Z have the abovementioned meaning,
are reacted with a hydrogenating agent, optionally in the presence of a reaction aid and optionally in the presence of a diluent,
or in that
(d) compounds of the general formula (I), in which R and R³ together represent alkoxyalkanediyl or dialkoxyalkanediyl and R¹, R⁴, Y and Z have the abovementioned meaning,
are reacted with a hydrogen halide and/or an alkali metal halide as well as in the presence of diluents,
or in that
(e) compounds of the general formula (I), in which R and R³ together represent hydroxyalkanediyl or dihydroxyalkanediyl and R¹, R⁴, Y and Z have the abovementioned meaning,
are reacted with an alkylating agent of the general formula (IV)
X¹-R (IV)
in which
R represents alkyl and
X¹ represents halogen or the grouping -O-SO₂-O-R in which R has the abovementioned meaning,
optionally in the presence of an acid binding agent and optionally in the presence of a diluent,
or in that
(f) compounds of the general formula (I), in which R⁴ represents hydrogen and R¹, R, R³, Y and Z have the abovementioned meaning,
are reacted with halogen compounds of the general formula (V)
X-R⁴ (V)
in which
X represents halogen and
R⁴ has, with the exception of hydrogen, the abovementioned meaning,
optionally in the presence of an acid acceptor and optionally in the presence of a diluent.

3. Substituted aza(cyclo)alkanes of the formula (I) according to Claim 1, in which
R¹ represents a five- or six-membered heterocyclic grouping from the series comprising pyrazoyl, 1,2,4-triazolyl, oxazolyl, isoxazolyl, thiazolyl, isothiazolyl, 1,2,5-thiadiazolyl, pyridyl, pyrazinyl and pyrimidinyl, which is substituted by fluorine, chlorine, bromine, cyano, nitro, C₁-C₂-alkyl (which is optionally substituted by fluorine and/or chlorine), C₁-C₂-alkoxy (which is optionally substituted by fluorine and/or chlorine), C₁-C₂-alkylthio (which is optionally substituted by fluorine and/or chlorine) or C₁-C₂-alkylsulphonyl (which is optionally substituted by fluorine and/or chlorine),
R represents C₁-C₄-hydroxyalkyl, dihydroxy-C₁-C₄-alkyl, C₁-C₃-alkoxy-C₁-C₃-alkyl or di- (C₁-C₃-alkoxy)-C₁-C₃-alkyl,
R³ represents hydrogen, C₁-C₄-alkyl, C₁-C₄-hydroxyalkyl, dihydroxy-C₁-C₄-alkyl, C₁-C₃-alkoxy-C₁-C₃-alkyl or di-(C₁-C₃-alkoxy)-C₁-C₃-alkyl or
R and R³ together represent hydroxy-C₂-C₃-alkanediyl, dihydroxy-C₂-C₃-alkanediyl, C₁-C₃-alkoxy-C₂-C₃-alkanediyl, di-(C₁-C₃-alkoxy)-C₂-C₃-alkanediyl, oxo-C₂-C₃-alkanediyl or dioxo-C₂-C₃-alkanediyl,
R⁴ represents hydrogen, methyl, ethyl, allyl, propargyl, formyl, C₁-C₈-alkylcarbonyl (which is optionally substituted by fluorine and/or chlorine), C₁-C₈-alkoxy-carbonyl, benzyloxycarbonyl, phenoxycarbonyl, benzyl (which is optionally substituted by fluorine or chlorine) or di-(C₁-C₂-alkoxy)-(thio)phosphoryl,
Y represents nitrogen or a CH grouping and
Z represents cyano or nitro.

4. Substituted aza(cyclo)alkanes of the formula (I) according to Claim 1, in which
R¹ represents 6-chloro-3-pyridyl (6-chloro-pyridin-3-yl) or represents 2-chloro-5-thiazolyl (2-chloro-thiazol-5-yl),
R represents hydroxymethyl, hydroxyethyl, hydroxypropyl, dihydroxyethyl, dihydroxypropyl, methoxymethyl, ethoxymethyl, methoxyethyl, ethoxyethyl, dimethoxyethyl or diethoxyethyl,
R³ represents hydrogen, methyl, ethyl, hydroxymethyl, hydroxyethyl, hydroxypropyl, dihydroxyethyl, dihydroxypropyl, methoxymethyl, ethoxymethyl, methoxyethyl, ethoxyethyl, dimethoxyethyl or diethoxyethyl,
R and R³ together represent oxoethane-1,2-diyl, dioxoethane-1,2-diyl, hydroxyethane-1,2-diyl, dihydroxyethane-1,2-diyl, methoxyethane-1,2-diyl, ethoxyethane-1,2-diyl, dimethoxyethane-1,2-diyl or diethoxyethane-1,2-diyl,
R⁴ represents hydrogen or methyl,
Y represents nitrogen or a CH grouping and
Z represents cyano or nitro.

5. Agents for combating pests, characterised by a content of at least one substituted aza(cyclo)alkane of the formula (I) according to Claim 1.

6. Use of substituted aza(cyclo)alkanes of the formula (I) according to Claim 1 for combating pests.

7. Process for combating pests, characterised in that substituted aza(cyclo)alkanes of the formula (I) according to Claim 1 are allowed to act on pests and/or their habitat.

8. Process for preparing agents for combating pests, characterised in that substituted aza(cyclo)alkanes of the formula (I) according to Claim 1 are mixed with extenders and/or surface-active agents.

## Revendications

1. Aza(cyclo)alcanes sustitués de formule générale (I) 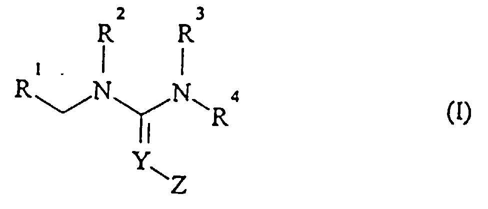 dans laquelle
R¹ représente un groupe hétérocyclique pentagonal ou hexagonal de la série furyle, thiényle, pyrrolyle, pyrazolyle, imidazolyle, 1,2,3- ou 1,2,4-triazolyle, oxazolyle, isoxazolyle, 1,2,4- ou 1,3,4-oxadiazolyle, thiazolyle, isothiazolyle, 1,2,3- 1,2,4-, 1,2,5- ou 1,3,4-thiadiazolyle, pyridyle, pyridazinyle, pyrimidinyle et pyrazinyle, qui peut être substitué par du fluor, du chlore, du brome, de l'iode, du groupe cyano, nitro, alkyle en C₁ à C₄ (qui est substitué le cas échéant par du fluor et/ou du chlore), un groupe alcényle en C₂ à C₄ (qui est substitué le cas échéant par du fluor et/ou du chlore), un groupe alcynyle en C₂ à C₄, un groupe alkoxy en C₁ à C₄ (qui est substitué le cas échéant par du fluor et/ou du chlore), un groupe alcényloxy en C₃ ou C₄ (qui est substitué le cas échéant par du fluor et/ou du chlore), un groupe alcynyloxy en C₃ ou C₄, un groupe alkylthio en C₃ ou C₄ (qui est substitué le cas échéant par du fluor et/ou du chlore), un groupe alcénylthio en C₃ ou C₄ (qui est substitué le cas échéant par du fluor et/ou du chlore), un groupe alcynylthio en C₃ ou C₄, un groupe alkylsulfinyle en C₁ à C₄ (qui est substitué le cas échéant par du fluor et/ou du chlore), un groupe alkylsulfonyle en C₁ à C₄ (qui est substitué le cas échéant par du fluor et/ou du chlore), un groupe amino, alkylamino en C₁ à C₄, di(alkyle en C₁ à C₄)amino, phényle, phénoxy, phénylthio, phénylamino, benzyle, formylamino, (alkyle en C₁ à C₄)carbonyramino, formyle, carbamoyle, (alkyle en C₁ à C₄)carbonyle et/ou (alkoxy en C₁ à C₄) carbonyle,
R est un groupe hydroxyalkyle en C₁ à C₆, dihydroxyalkyle en C₁ à C₆, (alkoxy en C₁ à C₄)-(alkyle en C₁ à C₄), di(alkoxy en C₁ à C₄)-(alkyle en C₁ à C₄),
R³ est de l'hydrogène, un groupe alkyle en C₁ à C₆, hydroxyalkyle en C₁ à C₆, dihydroxyalkyle en C₁ à C₆, (alkoxy en C₁ à C₄)-(alkyle en C₁ à C₄), di(alkoxy en C₁ à C₄)-(alkyle en C₁ à C₄), ou bien
R et R³ représentent ensemble un groupe hydroxyalcanediyle en C₂ à C₄, dihydroxyalcanediyle en C₂ à C₄, (alkoxy en C₁ à C₄)-(alcanediyle en C₂ à C₄), di(alkoxy en C₁ à C₄)-(alcanediyle en C₂ à C₄), oxy-(alcanediyle en C₂ à C₄) ou dioxo-(alcanediyle en C₂ à C₄),
R⁴ représente de l'hydrogène, un groupe alkyle en C₁ à C₄, (qui est substitué le cas échéant par du fluor, du chlore, un reste cyano, alkoxy en C₁ à C₄, alkylthio en C₁ à C₄, di(alkyle en C₁ à C₄)amino, triméthylsilyle, (alkoxy en C₁ à C₄)carbonyle, carboxy, carbamoyle, (alkyle en C₁ à C₄)aminocarbonyle, di(alkyle en C₁ à C₃)aminocarbonyle, ou par un groupement hétérocyclique tel que défini de préférence pour R¹ ci-dessus (y compris les substituants possibles), un groupe alcényle en C₂ à C₄, (qui est substitué le cas échéant par du fluor ou du chlore), un groupe alcynyle en C₂ à C₄, un groupe benzyle (qui est substitué le cas échéant par du fluor, du chlore, un reste cyano, nitro, alkyle en C₁ ou C₂, trifluorométhyle, alkoxy en C₁ ou C₂ ou (alkoxy en C₁ ou C₂)carbonyle, un groupe formyle, un groupe (alkyle en C₁ à C₂₀)carbonyle, (qui est substitué le cas échéant par du fluor, du chlore, du brome, un reste phényle, phénoxy ou alkoxy en C₁ à C₄), un groupe (cycloalkyle en C₃ à C₆)carbonyle (qui est substitué le cas échéant par du fluor, du chlore et/ou un reste alkyle en C₁ à C₄), un groupe (alcényle en C₂ à C₂₀)carbonyle, (qui est substitué le cas échéant par du fluor et/ou du chlore), un groupe phényl-carbonyle ou un groupe naphtylcarbonyle (qui sont substitués le cas échéant par du fluor, du chlore, du brome, un reste alkyle en C₁ à C₄, trifluorométhyle, cyano, nitro, alkoxy en C₁ à C₄ et/ou (alkoxy en C₁ à C₄)carbonyle), un groupe (alkoxy en C₁ à C₂₀)carbonyle, benzyloxycarbonyle, phénoxycarbonyle, (alkylthio en C₁ à C₄) carbonyle, benzylthiocarbonyle, phénylthiocarbonyle, (alkyle en C₁ à C₆)amino-carbonyle, di(alkyle en C₁ à C₄)amino-carbonyle, phénylaminocarbonyle, (qui est substitué le cas échéant par du fluor, du chlore, du brome, un reste cyano, nitro, alkyle en C₁ à C₄, trifluorométhyle, alkoxy en C₁ à C₄, fluoralkoxy en C₁ à C₂, chloro-fluoralkoxy en C₁ à C₂, alkylthio en C₁ à C₄, fluoralkylthio en C₁ ou C₂, chlorofluoralkylthio en C₁ ou C₂ ou (alkoxy en C₁ à C₄)carbonyle), un groupe benzoylaminocarbonyle (qui est substitué le cas échéant par du fluor, du chlore, du brome, un reste méthyle ou trifluorométhyle), un groupe phénylsulfonylaminocarbonyle (qui est substitué le cas échéant par du fluor, du chlore, du brome, un reste méthyle, trifluorométhyle, alkoxy en C₁ à C₄, fluoralkoxy en C₁ ou C₂, chlorofluoralkoxy en C₁ ou C₂ ou (alkoxy en C₁ à C₄)carbonyle), un groupe alkylthio en C₁ à C₄ (qui est substitué le cas échéant par du fluor et/ou du chlore), un groupe phénylthio (qui est substitué le cas échéant par du fluor, du chlore, du brome, un reste nitro ou méthyle), un groupe alkylsulfinyle en C₁ à C₄, un groupe alkylsulfonyle en C₁ à C₄ (qui est substitué le cas échéant par du fluor et/ou du chlore), un groupe phénylsulfinyle (qui est substitué le cas échéant par du fluor, du chlore, du brome, un reste nitro ou méthyle), un groupe phénylsulfonyle ou un groupe naphtylsulfonyle (qui sont substitués le cas échéant par du fluor, du chlore, du brome, un reste cyano, nitro, méthyle, trifluorométhyle, alkoxy en C₁ à C₄, fluoralkoxy en C₁ à C₂, chlorofluoralkoxy en C₁ ou C₂ et/ou (alkoxy en C₁ à C₄) carbonyle), un groupe diméthyl(thio)-phosphoryle, (alkyle en C₁ à C₄)-(alkoxy en C₁ à C₄)-(thio)-phosphoryle ou di(alkoxy en C₁ à C₄)-(thio)phosphoryle,
Y représente de l'azote ou un groupement CH et
Z est un groupe cyano ou nitro.

2. Procédé de production d'aza(cyclo)alcanes substitués de formule générale (I) 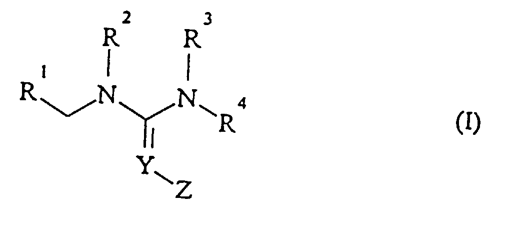 dans laquelle
R¹ représente un groupe hétérocyclique pentagonal ou hexagonal de la série furyle, thiényle, pyrrolyle, pyrazolyle, imidazolyle, 1,2,3- ou 1,2,4-triazolyle, oxazolyle, isoxazolyle, 1,2,4- ou 1,3,4-oxadiazolyle, thiazolyle, isothiazolyle, 1,2,3- 1,2,4-, 1,2,5- ou 1,3,4-thiadiazolyle, pyridyle, pyridazinyle, pyrimidinyle et pyrazinyle, qui peut être substitué par du fluor, du chlore, du brome, de l'iode, du groupe cyano, nitro, alkyle en C₁ à C₄ (qui est substitué le cas échéant par du fluor et/ou du chlore), un groupe alcényle en C₂ à C₄ (qui est substitué le cas échéant par du fluor et/ou du chlore), un groupe alcynyle en C₂ à C₄, un groupe alkoxy en C₁ à C₄ (qui est substitué le cas échéant par du fluor et/ou du chlore), un groupe alcényloxy en C₃ ou C₄ (qui est substitué le cas échéant par du fluor et/ou du chlore), un groupe alcynyloxy en C₃ ou C₄, un groupe alkylthio en C₃ ou C₄ (qui est substitué le cas échéant par du fluor et/ou du chlore), un groupe alcénylthio en C₃ ou C₄ (qui est substitué le cas échéant par du fluor et/ou du chlore), un groupe alcynylthio en C₃ ou C₄, un groupe alkylsulfinyle en C₁ à C₄ (qui est substitué le cas échéant du fluor et/ou du chlore), un groupe alkylsulfonyle en C₁ à C₄ (qui est substitué le cas échéant par du fluor et/ou du chlore), un groupe amino, alkylamino en C₁ à C₄, di(alkyle en C₁ à C₄)amino, phényle, phénoxy, phénylthio, phénylamino, benzyle, formylamino, (alkyle en C₁ à C₄)carbonylamino, formyle, carbamoyle, (alkyle en C₁ à C₄)carbonyle et/ou (alkoxy en C₁ à C₄)carbonyle,
R est un groupe hydroxyalkyle en C₁ à C₆, dihydroxyalkyle en C₁ à C₆, (alkoxy en C₁ à C₄)-(alkyle en C₁ à C₄), di(alkoxy en C₁ à C₄)-(alkyle en C₁ à C₄),
R³ est de l'hydrogène, un groupe alkyle en C₁ à C₆, hydroxyalkyle en C₁ à C₆, dihydroxyalkyle en C₁ à C₆, (alkoxy en C₁ à C₄)-(alkyle en C₁ à C₄), di(alkoxy en C₁ à C₄)-(alkyle en C₁ à C₄), ou bien
R et R³ représentent ensemble un groupe hydroxyalcanediyle en C₂ à C₄, dihydroxyalcanediyle en C₂ à C₄, (alkoxy en C₁ à C4)-(alcanediyle en C₂ à C₄), di(alkoxy en C₁ à C₄)-(alcanediyle en C₂ à C₄), oxy-(alcanediyle en C₂ à C₄) ou dioxo-(alcanediyle en C₂ à C₄),
R⁴ représente de l'hydrogène, un groupe alkyle en C₁ à C₄, (qui est substitué le cas échéant par du fluor, du chlore, un reste cyano, alkoxy en C₁ à C₄, alkylthio en C₁ à C₄, di(alkyle en C₁ à C₄)amino, triméthylsilyle, (alkoxy en C₁ à C₄)carbonyle, carboxy, carbamoyle, (alkyle en C₁ à C₄)aminocarbonyle, di(alkyle en C₁ à C₃)aminocarbonyle, ou par un groupement hétérocyclique tel que défini de préférence pour R¹ ci-dessus (y compris les substituants possibles), un groupe alcényle en C₂ à C₄, (qui est substitué le cas échéant par du fluor ou du chlore), un groupe alcynyle en C₂ à C₄, un groupe benzyle (qui est substitué le cas échéant par du fluor, du chlore, un reste cyano, nitro, alkyle en C₁ ou C₂, trifluorométhyle, alkoxy en C₁ ou C₂ ou (alkoxy en C₁ ou C₂)carbonyle, un groupe formyle, un groupe (alkyle en C₁ à C₂₀)carbonyle, (qui est substitué le cas échéant par du fluor, du chlore, du brome, un reste phényle, phénoxy ou alkoxy en C₁ à C₄), un groupe (cycloalkyle en C₃ à C₆)carbonyle (qui est substitué le cas échéant par du fluor, du chlore et/ou un reste alkyle en C₁ à C₄), un groupe (alcényle en C₂ à C₂₀)carbonyle, (qui est substitué le cas échéant par du fluor et/ou du chlore), un groupe phénylcarbonyle ou un groupe naphtylcarbonyle (qui sont substitués le cas échéant par du fluor, du chlore, du brome, un reste alkyle en C₁ à C₄, trifluorométhyle, cyano, nitro, alkoxy en C₁ à C₄ et/ou (alkoxy en C₁ à C₄)carbonyle), un groupe (alkoxy en C₁ à C₂₀)carbonyle, benzyloxycarbonyle, phénoxycarbonyle, (alkylthio en C₁ à C₄)carbonyle, benzylthiocarbonyle, phénylthiocarbonyle, (alkyle en C₁ à C₆)amino-carbonyle, di(alkyle en C₁ à C₄)amino-carbonyle, phénylaminocarbonyle, (qui est substitué le cas échéant par du fluor, du chlore, du brome, un reste cyano, nitro, alkyle en C₁ à C₄, trifluorométhyle, alkoxy en C₁ à C₄, fluoralkoxy en C₁ à C₂, chloro-fluoralkoxy en C₁ à C₂, alkylthio en C₁ à C₄, fluoralkylthio en C₁ ou C₂, chlorofluoralkylthio en C₁ ou C₂ ou (alkoxy en C₁ à C₄)carbonyle), un groupe benzoylaminocarbonyle (qui est substitué le cas échéant par du fluor, du chlore, du brome, un reste méthyle ou trifluorométhyle), un groupe phénylsulfonylaminocarbonyle (qui est substitué le cas échéant par du fluor, du chlore, du brome, un reste méthyle, trifluorométhyle, alkoxy en C₁ à C₄, fluoralkoxy en C₁ ou C₂, chlorofluoralkoxy en C₁ ou C₂ ou (alkoxy en C₁ à C₄)carbonyle), un groupe alkylthio en C₁ à C₄ (qui est substitué le cas échéant par du fluor et/ou du chlore), un groupe phénylthio (qui est substitué le cas échéant par du fluor, du chlore, du brome, un reste nitro ou méthyle), un groupe alkylsulfinyle en C₁ à C₄, un groupe alkylsulfonyle en C₁ à C₄ (qui est substitué le cas échéant par du fluor et/ou du chlore), un groupe phénylsulfinyle (qui est substitué le cas échéant par du fluor, du chlore, du brome, un reste nitro ou méthyle), un groupe phénylsulfonyle ou un groupe naphtylsulfonyle (qui sont substitués le cas échéant par du fluor, du chlore, du brome, un reste cyano, nitro, méthyle, trifluorométhyle, alkoxy en C₁ à C₄, fluoralkoxy en C₁ à C₂, chlorofluoralkoxy en C₁ ou C₂ et/ou (alkoxy en C₁ à C₄)carbonyle), un groupe diméthyl(thio)-phosphoryle, (alkyle en C₁ à C₄)-(alkoxy en C₁ à C₄)-(thio)-phosphoryle ou di(alkoxy en C₁ à C₄)-(thio)phosphoryle,
Y représente de l'azote ou un groupement CH et
Z est un groupe cyano ou nitro.
caractérisé en ce que :
(a) on fait réagir des azalcanes de formule générale (II) 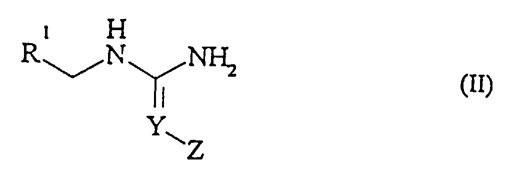 dans laquelle
R¹, Y et Z ont la définition indiquée ci-dessus, avec des composés halogénés de formule générale (IIIa)
X-R (IIIa)
et/ou des composés halogénés de formule générale (IIIb)
X-R³ (IIIb)
formules dans lesquelles
R et R³ ont la définition indiquée ci-dessus et
X représente un halogène,
le cas échéant en présence d'un accepteur d'acide, éventuellement en présence d'un catalyseur et en la présence éventuelle d'un diluant, ou bien
(b) on chauffe des composés de formule générale (I) dans laquelle R et R³ représentent un groupe dialkoxyalkyle et R¹, R⁴, Y et Z ont la définition indiquée ci-dessus,
éventuellement en présence d'un auxiliaire de réaction et en la présence éventuelle d'un diluant,
ou bien
(c) on fait réagir des composés de formule générale (I) dans laquelle R et R³ représentent ensemble un groupe oxoalcanediyle et R¹, R⁴, Y et Z ont la définition indiquée ci-dessus,
avec un agent hydrogénant, le cas échéant en présence d'un auxiliaire de réaction et en la présence éventuelle d'un diluant,
ou bien
(d) on fait réagir des composés de formule générale (I) dans laquelle R et R³ forment ensemble un groupe alkoxyalcanediyle ou dialkoxyalcanediyle et R¹, R⁴, Y et Z ont la définition indiquée ci-dessus,
avec un halogénure d'hydrogène et/ou un halogénure de métal alcalin ainsi qu'en présence de diluants,
ou bien
(e) on fait réagir des composés de formule générale (I) dans laquelle R et R³ forment ensemble un groupe hydroxyalcanediyle ou dihydroxyalcanediyle et R¹, R⁴, Y et Z ont la définition indiquée ci-dessus,
avec un agent alkylant de formule générale (IV)
X¹-R (IV)
dans laquelle
R est un groupe alkyle et
X¹ représente un halogène ou le groupe -O-SO₂-O-R dans lequel R a la définition indiquée ci-dessus,
éventuellement en présence d'un accepteur d'acide et en la présence éventuelle d'un diluant,
ou bien
(f) on fait réagir des composés de formule générale (I) dans laquelle R⁴ représente de l'hydrogène et R¹, R, R³, Y et Z ont la définition indiquée ci-dessus,
avec des composés halogénés de formule générale (V)
X-R⁴ (V)
dans laquelle
X est un halogène et
R⁴ a la définition indiquée ci-dessus, excepté l'hydrogène,
le cas échéant en présence d'un accepteur d'acide et en la présence éventuelle d'un diluant.

3. Aza(cyclo)alcanes substitués de formule (I) suivant la revendication 1, dans laquelle
R¹ représente un groupement hétérocyclique pentagonal ou hexagonal de la série pyrazolyle, 1,2,4-triazolyle, oxazolyle, isoxazolyle, thiazolyle, isothiazolyle, 1,2,5-thiadiazolyle, pyridyle, pyrazinyle et pyrimidinyle, qui est substitué par du fluor, du chlore, du brome, un reste cyano, nitro, alkyle en C₁ ou C₂ (qui est éventuellement substitué par du fluor et/ou du chlore), un groupe alkoxy en C₁ ou C₂ (qui est substitué le cas échéant par du fluor et/ou du chlore), un groupe alkylthio en C₁ ou C₂ (qui est substitué le cas échéant par du fluor et/ou du chlore) ou un groupe alkylsulfonyle en C₁ ou C₂ (qui est substitué le cas échéant par du fluor et/ou du chlore),
R est un groupe hydroxyalkyle en C₁ à C₄, dihydroxyalkyle en C₁ à C₄, (alkoxy en C₁ à C₃)-(alkyle en C₁ à C₃), di(alkoxy en C₁ à C₃)-(alkyle en C₁ à C₃),
R³ est de l'hydrogène, un groupe alkyle en C₁ à C₄, hydroxyalkyle en C₁ à C₄, dihydroxy(alkyle en C₁ à C₄), (alkoxy en C₁ à C₃)-(alkyle en C₁ à C₃), di(alkoxy en C₁ à C₃)-(alkyle en C₁ à C₃), ou bien
R et R³ forment ensemble un groupe hydroxyalcanediyle en C₂ ou C₃, dihydroxyalcanediyle en C₂ ou C₃ (alkoxy en C₁ à C₃)-(alcanediyle en C₂ ou C₃), di(alkoxy en C₁ à C₃)-(alcanediyle en C₂ ou C₃), oxo-alcanediyle en C₂ ou C₃ ou dioxo-alcanediyle en C₂ ou C₃,
R⁴ représente de l'hydrogène, un groupe méthyle, éthyle, allyle, propargyle, formyle, (alkyle en C₁ à C₈)carbonyle (qui est substitué le cas échéant par du fluor et/ou du chlore), un groupe (alkoxy en C₁ à C₈)carbonyle, benzyloxycarbonyle, phénoxycarbonyle, benzyle, (qui est substitué le cas échéant par du fluor ou du chlore) ou di(alkoxy en C₁ ou C₂) (thio)phosphoryle,
Y représente de l'azote ou un groupement CH et
Z est un groupe cyano ou nitro.

4. Aza(cyclo)alcanes substitués de formule (I) suivant la revendication 1, dans laquelle
R¹ est un groupe 6-chloro-3-pyridyle (6-chloropyridine-3-yle) ou un groupe 2-chloro-5-thiazolyle (2-chlorothiazole-5-yle),
R est un groupe hydroxyméthyle, hydroxyéthyle, hydroxypropyle, dihydroxyéthyle, dihydroxypropyle, méthoxyméthyle, éthoxyméthyle, méthoxyéthyle, éthoxyéthyle, diméthoxyéthyle, diéthoxyéthyle.
R³ est de l'hydrogène, un groupe méthyle, éthyle, hydroxyméthyle, hydroxyéthyle, hydroxypropyle, dihydroxyéthyle, dihydroxypropyle, méthoxyméthyle, éthoxyméthyle, méthoxyéthyle, éthoxyéthyle, diméthoxyéthyle, diéthoxyéthyle,
R et R³ forment ensemble un groupe oxoéthane-1,2-diyle, dioxoéthane-1,2-diyle, hydroxyéthane-1,2-diyle, dihydroxyéthane-1,2-diyle, méthoxyéthane-1,2-diyle, éthoxyéthane-1,2-diyle, diméthoxyéthane-1,2-diyle ou diéthoxyéthane-1,2-diyle,
R⁴ est de l'hydrogène ou un groupe méthyle,
Y est de l'azote ou un groupement CH et
Z est un groupe cyano ou nitro.

5. Compositions pesticides, caractérisées par une teneur en au moins un aza(cyclo)alcane substitué de formule (I) suivant la revendication 1.

6. Utilisation d'aza(cyclo)alcanes substitués de formule (I) suivant la revendication 1 pour combattre des parasites.

7. Procédé pour combattre des parasites, caractérisé en ce qu'on fait réagir des aza(cyclo)alcanes substitués de formule (I) suivant la revendication 1 sur les parasites et/ou sur leurs milieux.

8. Procédé de préparation de compositions pesticides, caractérisé en ce qu'on mélange des aza(cyclo)-alcanes substitués de formule (I) suivant la revendication 1 avec des diluants et/ou des agents tensioactifs.
